# EUROPEAN PATENT APPLICATION

(11) **EP 3 798 616 A1**
(43) Date of publication of application: **31.03.2021**
(21) Application number: 19807781.0
(22) Date of filing: 21.05.2019
(51) Int. Cl.: G01N 21/76

(54) **CHEMICAL LUMINESCENCE ANALYSIS AND MEASUREMENT METHOD, SYSTEM USING SAME, AND KIT**

(30) Priority: 21.05.2018 CN 201810491322
(71) Applicant: Beyond Diagnostics (Shanghai) Co., Ltd, Shanghai 201210 (CN); Chemclin Diagnostics Co., Ltd., Beijing 100094 (CN)
(72) Inventor: YANG, Yang, Shanghai 201210 (CN); ZHAO, Weiguo, Shanghai 201210 (CN); LIU, Yuhui, Beijing 100094 (CN); LI, Lin, Beijing 100094 (CN)
(74) Representative: Dr. Gassner & Partner mbB
(86) International application number: PCT/CN2019/087829
(87) International publication number: WO 2019/223692

(57) **Abstract**

A chemical luminescence immune analysis and measurement method, a system using the chemical luminescence immune analysis and measurement method, and a kit are disclosed. The method broadens a detection range and indicates an HD-HOOK sample or a sample that is beyond the detection range by way of selecting two signal values from signal values that are read in multiple times.

## Description

### Cross-reference to Related Applications

This application claims the priority to Chinese patent application CN 201810491322.9, entitled "Chemical luminescence analysis and measurement method, system using same, and kit" and filed on May 21, 2018, the entirety of which is incorporated herein by reference.

### Field of the Invention

The present disclosure relates to the field of chemiluminescence technology, in particular, to a chemical luminescence analysis and measurement method, a system using the chemical luminescence analysis and measurement method, and a kit.

### Background of the Invention

Chemiluminescent immunoassay is a non-radioactive immunoassay that has evolved rapidly in recent years. Its principle is to use a chemiluminescent substance(s) to magnify a signal associated directly with the binding of an antibody to an antigen, thus to detect the binding process through the luminescent intensity of the chemiluminescent substance(s). Chemiluminescent immunoassay has become one of the most important techniques in the field of immunoassay. Light excited chemiluminescent assay is a common technique applied in the field of chemiluminescent immunoassay. It can be used to study the interactions of biomolecules. Clinically, it is mostly utilized for the detection of diseases. Light excited chemiluminescent assay utilizes and integrates the knowledge of many related fields especially macromolecular particles, organic synthesis, protein chemistry, and clinical detection. Compared with traditional enzyme-linked immuno sorbent assay (ELISA), light excited chemiluminescent assay is homogeneous, more sensitive, easier to operate and more automatic, and thus has a prospect of being widely used.

In a double-antibody sandwich detection assay of the chemiluminescent immunoassay, when a concentration of an analyte is high to a certain value, no double-antibody sandwich complex can be formed and a low signal is thus observed. This phenomenon is called high dose hook effect (HD-HOOK effect). In other words, HD-HOOK effect refers to a phenomenon where in a double-site sandwich immunoassay, a high dose section of a dose response curve does not linearly extend backwards indefinitely like a platform, but bends downwards like a hook, in which case the result is a false negative. HD-HOOK effect occurs frequently in immunoassays, and its occurrence rate accounts for 30% of positive samples. Due to the existence of HD-HOOK effect, it cannot be determined whether a concentration of a sample to be detected has exceeded a linear range of an assay kit or the concentration of the sample is really the detected value, which results in experimental misdiagnosis, especially increase of occurrence rate of false negatives.

It is therefore urgently desirable to provide a chemical luminescence analysis and measurement method that can broaden the detection range and avoid the HD-HOOK effect.

### Summary of the Invention

Directed against the defects of the existing technologies, the present disclosure aims to provide a chemical luminescence immune analysis and measurement method. The method broadens a detection range and indicates an HD-HOOK sample or a sample that is beyond the detection range by way of selecting any two signal values from signal values that are read in multiple times.

In order to realize the above and related objectives, the present disclosure, according to a first aspect thereof, provides a chemical luminescence immune analysis and measurement method. The method includes the following steps.
(1) A sample to be detected suspected of containing target molecules to be detected is mixed and reacted with reagents required for a chemiluminescent immunoreaction to form a mixture to be detected.
(2) The mixture to be detected is excited successively t times to undergo chemiluminescence, and signal values with respect to the chemiluminescence are recorded n times. An n^{th}-time-recorded signal value with respect to the chemiluminescence is marked as RLUn.
(3) Any two signal values are selected from the signal values with respect to the chemiluminescence recorded in the n times, and are marked as RLUm and RLUk, respectively. A growth rate from RLUm to RLUk is marked as A.
(4) A standard curve is made based on a series of known concentrations of standard substances containing the target molecules to be detected as well as a growth rate A' from RLUm' to RLUk' read with respect to any two reactions of the standard substances happened in step (2) and step (3). The concentrations of the standard substances are lower than a concentration at which a HOOK effect occurs.
(5) If the growth rate A is greater than a maximum value of the standard curve, the sample to be detected is diluted and then measured again.

t, n, m, and k are natural numbers greater than 0, and k<m≤n≤t, n≥2.

In some embodiments of the present disclosure, the growth rate A=(RLUm/RLUk-1)×100%.

In some other embodiments of the present disclosure, n is greater than 2.

In some embodiments of the present disclosure, in step (1), the chemiluminescent immunoreaction is a homogeneous chemiluminescent immunoreaction.

In some preferred embodiments of the present disclosure, in step (1), the reagents required for the chemiluminescent immunoreaction include an acceptor reagent and a donor reagent.The donor reagent includes a donor which is capable of generating singlet oxygen in an excited state. The receptor reagent includes an acceptor which is capable of reacting with singlet oxygen to generate a detectable chemiluminescence signal value.

In some embodiments of the present disclosure, the acceptor is polymer particles filled with a light-emitting compound and a lanthanide compound.

In some preferred embodiments of the present disclosure, the light-emitting compound is selected from olefin compounds, preferably selected from thioxene, dibutanedione compounds, dioxene, enol ether, enamine, 9-alkylene xanthane, 9-alkylene-N-9,10 dihydroacridine, aryl ether alkene, aryl imidazole, lucigenin, and their derivatives, more preferably selected from thioxene and its derivatives.

In some other preferred embodiments of the present disclosure, the lanthanide compound is a europium complex.

In some embodiments of the present disclosure, the acceptor includes an olefin compound and a metal chelate which are in a non-particle form and are soluble in a water-bearing medium.

In some specific embodiments of the present disclosure, the acceptor binds directly or indirectly to a first binding conjugate specific to the target molecules to be detected.

In some embodiments of the present disclosure, the donor is polymer particles filled with a photosensitive compound which is capable of generating singlet oxygen when excited by a red laser beam.

In some other embodiments of the present disclosure, the photosensitive compound is selected from one of methylene blue, rose red, porphyrin, and phthalocyanine.

In some embodiments of the present disclosure, the donor binds directly or indirectly to a label.

In some preferred embodiments of the present disclosure, in step (1), the reagents required for the chemiluminescent immunoreaction further include a second binding conjugate specific to the target molecules to be detected. Preferably, the second binding conjugate specific to the target molecules to be detected binds directly or indirectly to a binding conjugate specific to the label.

In some embodiments of the present disclosure, in step (1), the sample to be detected containing the target molecules to be detected is first mixed with the acceptor reagent and the second binding conjugate specific to the target molecules to be detected, and then mixed with the donor reagent.

In some specific embodiments of the present disclosure, in step (2), the mixture to be detected is excited to undergo the chemiluminescence by means of energy and/or an active compound. Preferably, the mixture to be detected is irradiated with red excitation light of 600-700 nm so as to be excited to undergo the chemiluminescence.

In some other specific embodiments of the present disclosure, in step (2), a wavelength that is detected for recording the signal values with respect to the chemiluminescence is 520-620 nm.

In some embodiments of the present disclosure, the target molecules to be detected are an antigen or an antibody. The antigen is an immunogenic substance, and the antibody is an immunoglobulin which is produced by the body capable of recognizing a unique foreign substance.

In some other embodiments of the present disclosure, the standard substance is used as a positive control.

In some specific embodiments of the present disclosure, the method specifically includes the following steps.
(a1) A sample to be detected suspected of containing an antigen (or antibody) to be detected is mixed with an acceptor reagent, and subjected to a first incubation to obtain a mixed solution. The mixed solution obtained from the first incubation is then mixed with a donor reagent, and subjected to a second incubation to form a mixture to be detected.
(a2) The mixture to be detected is excited successively t times with red excitation light of 600-700 nm to undergo chemiluminescence, and signal values with respect to the chemiluminescence are recorded n times. A wavelength detected is 520-620 nm. An n^{th}-time-recorded signal value with respect to the chemiluminescence is marked as RLUn.
(a3) Any two signal values are selected from the signal values with respect to the chemiluminescence recorded in the n times, and are marked as RLUm and RLUk, respectively. A growth rate from RLUm to RLUk is marked as A. The growth rate A=(RLUm/RLUk-1)×100%.
(a4) A standard curve is made based on a series of known concentrations of positive controls containing target molecules to be detected as well as a growth rate A' from RLUm' to RLUk' read with respect to any two reactions of the positive controls happened in step (a2) and step (a3). The concentrations of the standard substances are lower than a concentration at which a HOOK effect occurs.
(a5) If the growth rate A is greater than a maximum value of the standard curve, the sample to be detected is diluted and then measured again.

t, n, m, and k are natural numbers greater than 0, and k<m≤n≤t, n≥2.

The present disclosure, according to a second aspect thereof, provides a system using a chemical luminescence immune analysis and measurement method. The system includes a reaction device, an excitation and reading device, and a processor.

The reaction device is configured to conduct a chemiluminescent immunoreaction.

The excitation and reading device is configured to: excite the mixture to be detected successively t times to cause the mixture to be detected to undergo chemiluminescence, and record n times signal values with respect to the chemiluminescence, an n^{th}-time-recorded signal value with respect to the chemiluminescence being marked as RLUn; and select any two signal values from the signal values with respect to the chemiluminescence recorded in the n times, mark the selected two signal values as RLUm and RLUk, respectively, and mark a growth rate from RLUm to RLUk as A.

The processor is configured to: make a standard curve based on a series of known concentrations of standard substances containing target molecules to be detected as well as a growth rate A' from RLUm' to RLUk' read with respect to any two reactions of the standard substances. The concentrations of the standard substances are lower than a concentration at which a HOOK effect occurs. If the growth rate A is greater than a maximum value of the standard curve, the sample to be detected is diluted and then measured again.

t, n, m, and k are natural numbers greater than 0, and k<m≤n≤t, n≥2.

In some specific embodiments of the present disclosure, a method of using the system includes the following steps.
(1) A sample to be detected suspected of containing target molecules to be detected is mixed and reacted with reagents required for a chemiluminescent immunoreaction to form a mixture to be detected.
(2) The mixture to be detected is excited successively t times to undergo chemiluminescence, and signal values with respect to the chemiluminescence are recorded n times. An n^{th}-time-recorded signal value with respect to the chemiluminescence is marked as RLUn.
(3) Any two signal values are selected from the signal values with respect to the chemiluminescence recorded in the n times, and are marked as RLUm and RLUk, respectively. A growth rate from RLUm to RLUk is marked as A.
(4) A standard curve is made based on a series of known concentrations of standard substances containing the target molecules to be detected as well as a growth rate A' from RLUm' to RLUk' read with respect to any two reactions of the standard substances happened in step (2) and step (3). The concentrations of the standard substances are lower than a concentration at which a HOOK effect occurs.
(5) If the growth rate A is greater than a maximum value of the standard curve, the sample to be detected is diluted and then measured again.

t, n, m, and k are natural numbers greater than 0, and k<m≤n≤t, n≥2.

In some embodiments of the present disclosure, the growth rate A=(RLUm/RLUk-1)×100%.

In some other embodiments of the present disclosure, n is greater than 2.

In some embodiments of the present disclosure, in step (1), the chemiluminescent immunoreaction is a homogeneous chemiluminescent immunoreaction.

In some preferred embodiments of the present disclosure, in step (1), the reagents required for the chemiluminescent immunoreaction include an acceptor reagent and a donor reagent. The donor reagent includes a donor which is capable of generating singlet oxygen in an excited state. The acceptor reagent includes an acceptor which is capable of reacting with singlet oxygen to generate a detectable chemiluminescence signal value.

In some embodiments of the present disclosure, the acceptor is polymer particles filled with a light-emitting compound and a lanthanide compound.

In some preferred embodiments of the present disclosure, the light-emitting compound is selected from olefin compounds, preferably selected from thioxene, dibutanedione compounds, dioxene, enol ether, enamine, 9-alkylene xanthane, 9-alkylene-N-9,10 dihydroacridine, aryl ether alkene, aryl imidazole, lucigenin, and their derivatives, more preferably selected from thioxene and its derivatives.

In some other preferred embodiments of the present disclosure, the lanthanide compound is a europium complex.

In some embodiments of the present disclosure, the acceptor includes an olefin compound and a metal chelate which are in a non-particle form and are soluble in a water-bearing medium.

In some specific embodiments of the present disclosure, the acceptor binds directly or indirectly to a first binding conjugate specific to the target molecules to be detected.

In some embodiments of the present disclosure, the donor is polymer particles filled with a photosensitive compound which is capable of generating singlet oxygen when excited by a red laser beam.

In some preferred embodiments of the present disclosure, the photosensitive compound is selected from one of methylene blue, rose red, porphyrin, and phthalocyanine.

In some embodiments of the present disclosure, the donor binds directly or indirectly to a label.

In some preferred embodiments of the present disclosure, in step (1), the reagents required for the chemiluminescent immunoreaction further include a second binding conjugate specific to the target molecules to be detected. Preferably, the second binding conjugate specific to the target molecules to be detected binds directly or indirectly to a binding conjugate specific to the label.

In some embodiments of the present disclosure, in step (1), the sample to be detected containing the target molecules to be detected is first mixed with the acceptor reagent and the second binding conjugate specific to the target molecules to be detected, and then mixed with the donor reagent.

In some embodiments of the present disclosure, in step (2), the mixture to be detected is excited to undergo the chemiluminescence by means of energy and/or an active compound. Preferably, the mixture to be detected is irradiated with red excitation light of 600-700 nm so as to be excited to undergo the chemiluminescence.

In some other embodiments of the present disclosure, in step (2), a wavelength that is detected for recording the signal values with respect to the chemiluminescence is 520-620 nm.

In some embodiments of the present disclosure, the target molecules to be detected are an antigen or an antibody. The antigen is an immunogenic substance, and the antibody is an immunoglobulin which is produced by the body capable of recognizing a unique foreign substance.

In some other embodiments of the present disclosure, the standard substance is used as a positive control.

In some specific preferred embodiments of the present disclosure, the method specifically includes the following steps.
(a1) A sample to be detected suspected of containing an antigen (or antibody) to be detected is mixed with an acceptor reagent, and subjected to a first incubation to obtain a mixed solution. The mixed solution obtained from the first incubation is then mixed with a donor reagent, and subjected to a second incubation to form a mixture to be detected.
(a2) The mixture to be detected is excited successively t times with red excitation light of 600-700 nm to undergo chemiluminescence, and signal values with respect to the chemiluminescence are recorded n times. A wavelength detected is 520-620 nm. An n^{th}-time-recorded signal value with respect to the chemiluminescence is marked as RLUn.
(a3) Any two signal values are selected from the signal values with respect to the chemiluminescence recorded in the n times, and are marked as RLUm and RLUk, respectively. A growth rate from RLUm to RLUk is marked as A. The growth rate A=(RLUm/RLUk-1)×100%.
(a4) A standard curve is made based on a series of known concentrations of positive controls containing target molecules to be detected as well as a growth rate A' from RLUm' to RLUk' read with respect to any two reactions of the positive controls happened in step (a2) and step (a3). The concentrations of the standard substances are lower than a concentration at which a HOOK effect occurs.
(a5) If the growth rate A is greater than a maximum value of the standard curve, the sample to be detected is diluted and then measured again.

t, n, m, and k are natural numbers greater than 0, and k<m≤n≤t, n≥2.

The present disclosure, according to a third aspect thereof, provides a kit which includes reagents required for chemical luminescence immune analysis and measurement. A method of using the kit includes the following steps.
(1) A sample to be detected suspected of containing target molecules to be detected is mixed and reacted with reagents required for a chemiluminescent immunoreaction to form a mixture to be detected.
(2) The mixture to be detected is excited successively t times to undergo chemiluminescence, and signal values with respect to the chemiluminescence are recorded n times. An n^{th}-time-recorded signal value with respect to the chemiluminescence is marked as RLUn.
(3) Any two signal values are selected from the signal values with respect to the chemiluminescence recorded in the n times, and are marked as RLUm and RLUk, respectively. A growth rate from RLUm to RLUk is marked as A.
(4) A standard curve is made based on a series of known concentrations of standard substances containing the target molecules to be detected as well as a growth rate A' from RLUm' to RLUk' read with respect to any two reactions of the standard substances happened in step (2) and step (3). The concentrations of the standard substances are lower than a concentration at which a HOOK effect occurs.
(5) If the growth rate A is greater than a maximum value of the standard curve, the sample to be detected is diluted and then measured again.

t, n, m, and k are natural numbers greater than 0, and k<m≤n≤t, n≥2.

In some specific embodiments of the present disclosure, the method of using the kit specifically includes the following steps.
(a1) A sample to be detected suspected of containing an antigen (or antibody) to be detected is mixed with an acceptor reagent, and subjected to a first incubation to obtain a mixed solution. The mixed solution obtained from the first incubation is then mixed with a donor reagent, and subjected to a second incubation to form a mixture to be detected.
(a2) The mixture to be detected is excited successively t times with red excitation light of 600-700 nm to undergo chemiluminescence, and signal values with respect to the chemiluminescence are recorded n times. A wavelength detected is 520-620 nm. An n^{th}-time-recorded signal value with respect to the chemiluminescence is marked as RLUn.
(a3) Any two signal values are selected from the signal values with respect to the chemiluminescence recorded in the n times, and are marked as RLUm and RLUk, respectively. A growth rate from RLUm to RLUk is marked as A. The growth rate A=(RLUm/RLUk-1)×100%.
(a4) A standard curve is made based on a series of known concentrations of standard substances containing target molecules to be detected as well as a growth rate A' from RLUm' to RLUk' read with respect to any two reactions of the standard substances happened in step (2) and step (3). The concentrations of the standard substances are lower than a concentration at which a HOOK effect occurs.
(a5) If the growth rate A is greater than a maximum value of the standard curve, the sample to be detected is diluted and then measured again.

t, n, m, and k are natural numbers greater than 0, and k<m≤n≤t, n≥2.

In some embodiments of the present disclosure, the growth rate A=(RLUm/RLUk-1)×100%.

In some other embodiments of the present disclosure, n is greater than 2.

The present disclosure, according to a fourth aspect thereof, provides use of the method according to the first aspect thereof, the system according to the second aspect thereof, or the kit according to the third aspect thereof, in AFP detection.

The present disclosure brings the following beneficial effects.
(1) The method of the present disclosure broadens the detection range and indicates an HD-HOOK sample or a sample that is beyond the detection range by way of selecting two signal values from signal values that are read in multiple times.
(2) The method of the present disclosure can accurately identify an HD-HOOK effect sample in double-antibody sandwich assay. The method can significantly improve the accuracy of double-antibody sandwich immunoassay and decrease false negative rate in double-antibody sandwich immunoassay.

### Brief Description of the Drawings

The present disclosure will be further described below in conjunction with the drawings.
Fig. 1 is a standard curve of concentrations and corresponding signal values in an embodiment.
Fig. 2 is a standard curve of concentrations and growth rates A between signal values read in multiple times in an embodiment.
Fig. 3 is a graph showing a relationship between a signal value obtained by detecting HCG+β using a method of the present disclosure and a concentration of a sample as well as a relationship between a growth rate A and the concentration of the sample.
Fig. 4 is a graph showing a relationship between a signal value obtained by detecting Ferr using a method of the present disclosure and a concentration of a sample as well as a relationship between a growth rate A and the concentration of the sample.
Fig. 5 is a graph showing a relationship between a signal value obtained by detecting anti-HIV using a method of the present disclosure and a concentration of a sample as well as a relationship between a growth rate A and the concentration of the sample.
Fig. 6 is a graph showing a relationship between a signal value obtained by detecting MYO using a method of the present disclosure and a concentration of a sample as well as a relationship between a growth rate A and the concentration of the sample.
Fig. 7 is a graph showing a relationship between a signal value obtained by detecting NT-proBNP using a method of the present disclosure and a concentration of a sample as well as a relationship between a growth rate A and the concentration of the sample.
Fig. 8 is a graph showing a relationship between a signal value obtained by detecting PCT using a method of the present disclosure and a concentration of a sample as well as a relationship between a growth rate A and the concentration of the sample.
Fig. 9 is a graph showing a relationship between a signal value obtained by detecting cTnI using a method of the present disclosure and a concentration of a sample as well as a relationship between a growth rate A and the concentration of the sample.

### Detailed Description of the Embodiments

A detailed description of the present disclosure will be provided below to make the present disclosure easy to understand. However, before the detailed description of the present disclosure, it shall be appreciated that the present disclosure is not limited to specific embodiments described herein. It shall also be appreciated that terms used herein are only intended for facilitating description of the specific embodiments, and are not meant to be restrictive.

Where a range of values is provided, it shall be appreciated that every intermediate value between an upper limit and a lower limit of the range and between other stipulated values or intermediate values in the specified range is included in the present disclosure. Upper limits and lower limits of these smaller ranges may be independently included in the smaller ranges and are also included in the present disclosure, subject to any explicitly excluded limit in the specified range. When the specified range includes one or two limits, a range excluding either or both of those included limits is also included in the present disclosure.

Unless otherwise defined, all terms used herein have the same meanings as those understood by one skilled in the art. Although any methods or materials similar or equivalent to those described herein may also be used in implementing of or testing the present disclosure, preferred methods and materials are now described.

Unless otherwise defined, all experiment methods, detection methods, and preparation methods used in the present disclosure are commonly used molecular biological, biochemical, chromatin structural and analytical, cell culturing, and recombinant DNA techniques in the art, as well as common techniques used in related fields. These techniques have been detailed in existing literature. Reference may be made to: Sambrook et al, MOLECULAR CLONING: A LABORATORY MANUAL, Second edition, Cold Spring Harbor Laboratory Press, 1989 and Third edition, 2001; Ausubel et al, CURRENT PROTOCOLS IN MOLECULAR BIOLOGY, John Wiley & Sons, New York, 1987 and periodic updates; the series METHODS IN ENZYMOLOGY, Academic Press, San Diego; Wolffe, CHROMATIN STRUCTURE AND FUNCTION, Third edition, Academic Press, San Diego, 1998; METHODS IN ENZYMOLOGY, Vol. 304, Chromatin (P.M.Wassarman and A.P.Wolffe, eds.), Academic Press, San Diego, 1999; METHODS IN MOLECULAR BIOLOGY, Vol. 119, Chromatin Protocols (P.B.Becker, ed.), Humana Press, Totowa, 1999, etc.

### I. Terms

The term "chemical luminescence immune analysis and measurement" as used in the present disclosure has the following meaning. A chemical immunoreaction can produce an electronically excited product, and when molecules of this product undergo radiative transition or transfer energy to other molecules that can emit light to cause the latter to undergo radiative transition, luminescence occurs. This phenomenon in which molecules are electronically excited due to the absorption of chemical energy and thus emit light is called chemiluminescence. The method of using chemiluminescence for chemical immune analysis and measurement of an analyte is called a chemical luminescence immune analysis and measurement method. The chemical luminescence immune analysis and measurement method can be not only a heterogeneous chemical luminescence immune analysis and measurement method, but also a homogeneous chemical luminescence immune analysis and measurement method.

The term "target molecules to be detected" as used in the present disclosure are immune molecules, such as an antigen or an antibody. The term "sample to be detected" as used in the present disclosure includes the target molecules to be detected. The term "mixture to be detected" in the present disclosure includes the sample to be detected.

The term "reagents required for chemical luminescence immune analysis and measurement" as used in the present disclosure refers to reagents required for a chemical immunoreaction to produce chemiluminescence. The following conditions must be met for a chemical immunoreaction to produce chemiluminescence. First, sufficient excitation energy must be provided by a separate step in the reaction, because energy released by a previous reaction will disappear in the solution due to vibration relaxation and thus light emission cannot be caused. Second, there has to be a favorable reaction process that enables energy released by the chemical immunoreaction to be accepted by at least one substance and causes the substance to be in an excited state. Third, molecules of the excited substance must have a certain chemiluminescence quantum yield so as to release photons, or be able to transfer their energy to other molecules to enable the latter to be in an excited state and thus release photons.

The reagents required for chemical luminescence immune analysis and measurement include but are not limited to the following substances: (1) reactants in a chemiluminescent immunoreaction; (2) a catalyst, a sensitizer or an inhibitor in a chemiluminescent immunoreaction; and (3) a reactant, a catalyst, a sensitizer, etc. in a coupling reaction.

The term "HOOK effect" as used in the present disclosure refers to the phenomenon where in a double-antibody sandwich assay, when a concentration of an analyte is high to a certain value, no double-antibody sandwich complex may be formed and a low signal is thus observed. In other words, HD-HOOK effect refers to the phenomenon where in a double-site sandwich immunological experiment, a high dose section of a dose response curve does not linearly extend backwards indefinitely like a platform, but bends downwards like a hook, in which case a false negative may be produced.

The term "successively" as used in the present disclosure is a time feature, which means that each of the "excitation" in multiple times is distinguishable by time unit.

The term "antibody" as used in the present disclosure is used in the broadest sense, including antibodies of any isotype, antibody fragments that retain specific binding to antigens, including but not limited to Fab, Fv, scFv, and Fd fragments, chimeric antibodies, humanized antibodies, single-chain antibodies, bispecific antibodies, and fusion proteins containing an antigen-binding portion of an antibody and non-antibody proteins. In any case, an antibody may be further conjugated with other moieties, such as biotin, streptavidin, etc.

The term "antigen" as used in the present disclosure refers to an immunogenic substance, such as protein and polypeptide. Typical antigens include (but are not limited to): cytokines, tumor markers, metalloproteins, cardiovascular diabetes related proteins, etc. The term "tumor marker" refers to a class of substances that are produced by tumor cells themselves or by the body in response to the tumor cells during the development and proliferation of tumors, reflecting the existence and growth of the tumors. Typical tumor markers in the art include (but are not limited to): alpha-fetoprotein (AFP), cancer antigen 125 (CA125), etc.

The term "binding" as used in the present disclosure refers to a direct combination between two molecules due to, for example, covalent, electrostatic, hydrophobic, ionic and/or hydrogen bonding interactions, including but not limited to interactions such as salt bridges and water bridges.

The term "specific binding" as used in the present disclosure refers to a mutual identification and a selective binding reaction between two substances. From the perspective of three-dimensional structures, it is the conformational correspondence between corresponding reactants.

The term "biotin" as used in the present disclosure is widely present in animal and plant tissues. There are two ring structures on molecules of biotin, namely an imidazolone ring and a thiophene ring, among which the imidazolone ring is a main part that binds to streptavidin. Activated biotin may be coupled with almost all known biological macromolecules (including proteins, nucleic acids, polysaccharides, lipids, etc.) under the mediation of a protein cross-linking agent. "Streptavidin" is a protein secreted by streptomycetes and has a molecular weight of 65kD. A "streptavidin" molecule consists of 4 identical peptide chains, each of which may bind to a biotin molecule. Therefore, each antigen or antibody may be coupled with multiple biotin molecules at the same time, thereby producing a "tentacle effect" and thus improves analysis sensitivity.

In any case, any reagents used in the present disclosure, including antigens, antibodies, acceptors or donors, may be conjugated with biotin or streptavidin depending on actual needs.

The term "donor" as used in the present disclosure refers to a sensitizer that, after be activated by energy or a reactive compound, may produce a reactive intermediate such as singlet oxygen that reacts with an acceptor. A donor may be photoactivated (such as dyes and aromatic compounds) or chemically activated (such as enzymes, metal salts, etc.).

In some specific embodiments of the present disclosure, the donor is a photosensitizer, which may be a photosensitizer known in the art or preferably a compound that is relatively light stable and does not react effectively with singlet oxygen. Non-limiting examples include compounds such as methylene blue, rose bengal, porphyrin, phthalocyanine, chlorophyll, and derivatives of these compounds having 1-50 atom substituents which are used to make these compounds more lipophilic or more hydrophilic, and/or as linking groups to specific binding and pairing members, as disclosed in U.S. Patent No. 5,709,994 (this patent document is hereby incorporated by reference in its entirety). Examples of other photo sensitizers known to those skilled in the art may also be used in the present disclosure, such as those described in U.S. Patent. No. 6,406,913 which is incorporated herein by reference.

In other specific embodiments of the present disclosure, the donor is other chemically activated sensitizers. Non-limiting examples of the donor are certain compounds that catalyze the conversion of hydrogen peroxide into singlet oxygen and water. Other examples of the donor include: 1,4-dicarboxyethyl-1,4-naphthalene endoperoxide, 9,10-diphenylanthracene-9,10-endoperoxide, etc.; heating these compounds enables these compounds to absorb light or these compounds themselves can directly absorb light and then release singlet oxygen.

The term "acceptor" as used in the present disclosure refers to a compound that can react with singlet oxygen to generate a detectable signal. The donor is activated by energy or an active compound and releases high-energy singlet oxygen. The high-energy singlet oxygen is then captured by a near-by acceptor. Thus, energy is transferred to activate the acceptor.

In some specific embodiments of the present disclosure, the acceptor is such a substance that undergoes a chemical reaction with singlet oxygen to form an unstable metastable intermediate which can break down and meanwhile emit light or emit light later. Typical examples of such substances include but are not limited to: enol ether, enamine, 9-alkylidene xanthan gum, 9-alkylidene-N-alkyl acridinium, vinylarylene ether, diepoxyethylene, thioxene, aromatic imidazole or lucigenin.

In other specific embodiments of the present disclosure, the acceptor may be: alkenes capable of reacting with singlet oxygen to form hydroperoxides or dioxetane that can break down into ketones or a carboxylic acid derivative; stable dioxetane that can break down under the action of light; acetylenes that can react with singlet oxygen to form diketones; hydrazones or hydrazides that can form azo compounds or azocarbonyl compounds, such as luminol; and aromatic compounds that can form endoperoxides. Specific, non-limiting examples of acceptors that can be used in accordance with the present disclosure and the claimed invention are described in U.S. Patent No. 5,340,716 (this patent document is incorporated herein by reference in its entirety).

In other specific embodiments of the present disclosure, the "donor" and/or "acceptor" may be embedded in a marix by a functional group to form "donor microspheres" and/or "acceptor microspheres". The term "matrix" as used in the present disclosure is microspheres or particles known to those skilled in the art, which may be of any size, be organic or inorganic, expandable or non-expandable, porous or non-porous, have any density but preferably have a density close to that of water, and preferably may float in water and be made of a transparent, partially transparent or opaque material. The matrix may be charged or not charged, and when charged, it is preferably negatively charged. The matrix may be solid (such as polymers, metals, glass, organic and inorganic substances such as minerals, salts and diatoms), small oil droplets (such as hydrocarbons, fluorocarbons, siliceous fluids), vesicles (which are for example synthetic, such as phospholipids, or natural, such as cells, and cell organs). The matrix may be latex particles or other particles containing organic or inorganic polymers, lipid bilayers such as liposomes, phospholipid vesicles, small oil droplets, silicon particles, metal sols, cells and microcrystalline dyes. The matrix is generally versatile or capable of binding to a donor or an acceptor through specific or non-specific covalent or non-covalent interactions. Many functional groups are available or may be incorporated. Typical functional groups include carboxylic acid, acetaldehyde, amino, cyano, vinyl, hydroxyl, sulfhydryl and the like. A non-limiting example of the matrix suitable for use in the present disclosure is carboxyl modified latex particles. Details of such matrix can be found in U.S. Patent No. 5,709,994 and U.S. Patent No. 5,780,646 (these two patent documents are hereby incorporated by reference in their entirety).

### II. Specific Embodiments

Basic principles of double-antibody sandwich assay:
Basic principles of double-antibody sandwich assay are well-known to those skilled in the art. A conventional process of a double-antibody sandwich assay includes: fixing a primary antibody to a solid-phase carrier; subjecting the primary antibody to a reaction with an antigen and then to a reaction with a labeled second antibody; and finally performing a chemiluminescent reaction or enzyme-linked immune sorbent assay to detect a signal.

The present disclosure will be described in detail below.

An immune measurement method according to a first aspect of the present disclosure includes the following steps.
(1) A sample to be detected suspected of containing target molecules to be detected is mixed and reacted with reagents required for a chemiluminescent immunoreaction to form a mixture to be detected.
(2) The mixture to be detected is excited successively t times to undergo chemiluminescence, and signal values with respect to the chemiluminescence are recorded n times. An n^{th}-time-recorded signal value with respect to the chemiluminescence is marked as RLUn.
(3) Any two signal values are selected from the signal values with respect to the chemiluminescence recorded in the n times and are marked as RLUm and RLUk, respectively. A growth rate from RLUm to RLUk is marked as A.
(4) A standard curve is made based on a series of known concentrations of standard substances containing the target molecules to be detected as well as a growth rate A' from RLUm' to RLUk' read with respect to any two reactions of the standard substances happened in step (2) and step (3). The concentrations of the standard substances are lower than a concentration at which the HOOK effect occurs.
(5) If the growth rate A is greater than a maximum value of the standard curve, then the sample to be detected is diluted and measured again.

t, n, m, and k are all natural numbers greater than 0, and k<m≤n≤t, n≥2.

In some embodiments of the present disclosure, the growth rate A=(RLUm/RLUk-1)×100%.

In other embodiments of the present disclosure, n is greater than 2. For example, n can be 3, 4 or 5 etc. In the present disclosure, when n is greater than 2, the method exhibits relatively high detection sensitivity and strong anti-HD-HOOK-effect capability.

In some embodiments of the present disclosure, in step (1), the chemiluminescent immunoreaction is a homogeneous chemiluminescent immunoreaction.

In some preferred embodiments of the present disclosure, in step (1), the reagents required for the chemiluminescent immunoreaction include an acceptor reagent and a donor reagent.

The donor reagent includes a donor which is capable of generating singlet oxygen in an excited state.

The acceptor reagent includes an acceptor which is capable of reacting with singlet oxygen to generate a detectable chemiluminescence signal value.

In some embodiments of the present disclosure, the acceptor is polymer particles filled with a light-emitting compound and a lanthanide compound.

In some preferred embodiments of the present disclosure, the light-emitting compound is selected from olefin compounds, preferably selected from thioxene, dibutanedione compounds, dioxene, enol ether, enamine, 9-alkylene xanthane, 9-alkylene-N-9,10 dihydroacridine, aryl ether alkene, aryl imidazole, lucigenin, and their derivatives, more preferably selected from thioxene and its derivatives.

In other preferred embodiments of the present disclosure, the lanthanide compound is a europium complex.

In some embodiments of the present disclosure, the acceptor includes an olefin compound and a metal chelate which are in a non-particle form and are soluble in a water-bearing medium.

In some specific embodiments of the present disclosure, the acceptor binds directly or indirectly to a first binding conjugate specific to the target molecules to be detected.

In some embodiments of the present disclosure, the donor is polymer particles filled with a photosensitive compound which is capable of generating singlet oxygen when excited by a red laser beam.

In other embodiments of the present disclosure, the photosensitive compound is one selected from methylene blue, rose red, porphyrin, and phthalocyanine.

In some embodiments of the present disclosure, the donor binds directly or indirectly to a label.

In some preferred embodiments of the present disclosure, in step (1), the reagents required for the chemiluminescent immunoreaction further include a second binding conjugate specific to the target molecules to be detected. Preferably, the second binding conjugate specific to the target molecules to be detected binds directly or indirectly to a binding conjugate specific to the label.

In some embodiments of the present disclosure, in step (1), the sample to be detected containing the target molecules to be detected is first mixed with the acceptor reagent and the second binding conjugate reagent specific to the target molecules to be detected, and then mixed with the donor reagent.

In some specific embodiments of the present disclosure, in step (2), the mixture to be detected is excited to undergo the chemiluminescence by means of energy and/or an active compound. Preferably, the mixture to be detected is irradiated with red excitation light of 600-700 nm so as to be excited to undergo the chemiluminescence.

In some other specific embodiments of the present disclosure, in step (2), a wavelength that is detected for recording the signal values with respect to the chemiluminescence is 520-620 nm.

In some embodiments of the present disclosure, the target molecules to be detected are an antigen or an antibody. The antigen is an immunogenic substance, and the antibody is an immunoglobulin which is produced by the body capable of recognizing a unique foreign substance.

In some preferred embodiments of the present disclosure, the standard substance is used as a positive control.

In some preferred embodiments of the present disclosure, the method specifically includes the following steps.
(a1) A sample to be detected suspected of containing an antigen (or antibody) to be detected is mixed with an acceptor reagent and then subjected to a first incubation. A mixed solution obtained from the first incubation is then mixed with a donor reagent and subjected to a second incubation to form a mixture to be detected.
(a2) The mixture to be detected is excited successively t times with red excitation light of 600-700 nm to undergo chemiluminescence, and signal values with respect to the chemiluminescence are recorded n times. A wavelength detected is 520-620 nm. An n^{th}-time-recorded signal value with respect to the chemiluminescence is marked as RLUn.
(a3) Any two signal values are selected from the signal values with respect to the chemiluminescence recorded in the n times and are marked as RLUm and RLUk, respectively. A growth rate from RLUm to RLUk is marked as A. The growth rate A=(RLUm/RLUk-1)×100%.
(a4) A standard curve is made based on a series of known concentrations of positive controls containing target molecules to be detected as well as a growth rate A' from RLUm' to RLUk' read with respect to any two reactions of the positive controls happened in step (a2) and step (a3). The concentrations of the standard substances are lower than a concentration at which the HOOK effect occurs.
(a5) If the growth rate A is greater than a maximum value of the standard curve, then the sample to be detected is diluted and measured again.

t, n, m, and k are natural numbers greater than 0, and k<m≤n≤t, n≥2.

A system using a chemical luminescence immune analysis and measurement method according to a second aspect of the present disclosure includes a reaction device, an excitation and reading device, and a processor.

The reaction device is configured to conduct a chemiluminescent immunoreaction.

The excitation and reading device is configured to excite the mixture to be detected successively t times to enable the mixture to be detected to undergo chemiluminescence, record signal values with respect to the chemiluminescence n times, select any two signal values from the signal values with respect to the chemiluminescence recorded in the n times, mark the selected two signal values as RLUm and RLUk, respectively, and mark a growth rate from RLUm to RLUk as A.

The processor is configured to make a standard curve based on a series of known concentrations of standard substances containing the target molecules to be detected as well as a growth rate A' from RLUm' to RLUk' read with respect to any two reactions of the standard substances. The concentrations of the standard substances are lower than a concentration at which the HOOK effect occurs. If the growth rate A is greater than a maximum value of the standard curve, then the sample to be detected is diluted and measured again.

t, n, m, and k are natural numbers greater than 0, and k<m≤n≤t, n≥2.

In some embodiments of the present disclosure, a method of using the system includes the following steps.
(1) A sample to be detected suspected of containing target molecules to be detected is mixed and reacted with reagents required for a chemiluminescent immunoreaction to form a mixture to be detected.
(2) The mixture to be detected is excited to undergo chemiluminescence successively t times, and signal values with respect to the chemiluminescence are recorded n times. An n^{th}-time-recorded signal value with respect to the chemiluminescence is marked as RLUn.
(3) Any two signal values are selected from the signal values with respect to the chemiluminescence recorded in the n times and are marked as RLUm and RLUk, respectively. A growth rate from RLUm to RLUk is marked as A.
(4) A standard curve is made based on a series of known concentrations of standard substances containing the target molecules to be detected as well as a growth rate A' from RLUm' to RLUk' read with respect to any two reactions of the standard substances happened in step (2) and step (3). The concentrations of the standard substances are lower than a concentration at which the HOOK effect occurs.
(5) If the growth rate A is greater than a maximum value of the standard curve, then the sample to be detected is diluted and measured again.

t, n, m, and k are natural numbers greater than 0, and k<m≤n≤t, n≥2.

In some embodiments of the present disclosure, the growth rate A=(RLUm/RLUk-1)×100%.

In other embodiments of the present disclosure, n is greater than 2. For example, n is 3, 4 or 5 etc. In the present disclosure, when n is greater than 2, the system exhibits relatively high detection sensitivity and strong anti-HD-HOOK-effect capability.

In some embodiments of the present disclosure, in step (1), the chemiluminescent immunoreaction is a homogeneous chemiluminescent immunoreaction.

In some preferred embodiments of the present disclosure, in step (1), the reagents required for the chemiluminescent immunoreaction include an acceptor reagent and a donor reagent. The donor reagent includes a donor which is capable of generating singlet oxygen in an excited state. The acceptor reagent includes an acceptor which is capable of reacting with singlet oxygen to generate a detectable chemiluminescence signal value.

In some embodiments of the present disclosure, the acceptor is polymer particles filled with a light-emitting compound and a lanthanide compound.

In some preferred embodiments of the present disclosure, the light-emitting compound is selected from olefin compounds, preferably selected from thioxene, dibutanedione compounds, dioxene, enol ether, enamine, 9-alkylene xanthane, 9-alkylene-N-9,10 dihydroacridine, aryl ether alkene, aryl imidazole, lucigenin, and their derivatives, more preferably selected from thioxene and its derivatives.

In some other preferred embodiments of the present disclosure, the lanthanide compound is a europium complex.

In some embodiments of the present disclosure, the acceptor includes an olefin compound and a metal chelate which are in a non-particle form and are soluble in a water-bearing medium.

In some specific embodiments of the present disclosure, the acceptor binds directly or indirectly to a first binding conjugate specific to the target molecules to be detected.

In some embodiments of the present disclosure, the donor is polymer particles filled with a photosensitive compound which is capable of generating singlet oxygen when excited by a red laser beam.

In some preferred embodiments of the present disclosure, the photosensitive compound is one selected from methylene blue, rose red, porphyrin, and phthalocyanine.

In some embodiments of the present disclosure, the donor binds directly or indirectly to a label.

In some preferred embodiments of the present disclosure, in step (1), the reagents required for the chemiluminescent immunoreaction further include a second binding conjugate specific to the target molecules to be detected. Preferably, the second binding conjugate specific to the target molecules to be detected binds directly or indirectly to a binding conjugate specific to the label.

In some embodiments of the present disclosure, in step (1), the sample to be detected containing the target molecules to be detected is first mixed with the acceptor reagent and the second binding conjugate reagent specific to the target molecules to be detected, and then mixed with the donor reagent.

In some embodiments of the present disclosure, in step (2), the mixture to be detected is excited to undergo the chemiluminescence by means of energy and/or an active compound. Preferably, the mixture to be detected is irradiated with red excitation light of 600-700 nm so as to be excited to undergo the chemiluminescence.

In other embodiments of the present disclosure, in step (2), a wavelength that is detected for recording the signal values with respect to the chemiluminescence is 520-620 nm.

In some embodiments of the present disclosure, the target molecules to be detected are an antigen or an antibody. The antigen is an immunogenic substance, and the antibody is an immunoglobulin which is produced by the body capable of recognizing a unique foreign substance.

In some preferred embodiments of the present disclosure, the standard substance is used as a positive control.

In some preferred specific embodiments of the present disclosure, a method of using the system specifically includes the following steps.
(a1) The sample to be detected suspected of containing the antigen (or antibody) to be detected is mixed with the acceptor reagent and then subjected to a first incubation. A mixed solution obtained from the first incubation is then mixed with the donor reagent and subjected to a second incubation to form the mixture to be detected.
(a2) The mixture to be detected is excited successively t times with red excitation light of 600-700 nm to undergo chemiluminescence, and signal values with respect to the chemiluminescence are recorded n times. A wavelength detected is 520-620 nm. An n^{th}-time-recorded signal value with respect to the chemiluminescence is marked as RLUn.
(a3) Any two signal values are selected from the signal values with respect to the chemiluminescence recorded in the n times and are marked as RLUm and RLUk, respectively. A growth rate from RLUm to RLUk is marked as A. The growth rate A=(RLUm/RLUk-1)×100%.
(a4) A standard curve is made based on a series of known concentrations of positive controls containing target molecules to be detected as well as a growth rate A' from RLUm' to RLUk' read with respect to any two reactions of the positive controls happened in step (a2) and step (a3). The concentrations of the standard substances are lower than a concentration at which the HOOK effect occurs.
(a5) If the growth rate A is greater than a maximum value of the standard curve, then the sample to be detected is diluted and measured again

t, n, m, and k are natural numbers greater than 0, and k<m≤n≤t, n≥2.

A kit according to a third aspect of the present disclosure includes reagents required for chemical luminescence immune analysis and measurement. A method of using the kit includes the following steps.
(1) A sample to be detected suspected of containing target molecules to be detected is mixed and reacted with reagents required for a chemiluminescent immunoreaction to form a mixture to be detected.
(2) The mixture to be detected is excited to undergo chemiluminescence successively t times, and signal values with respect to the chemiluminescence are recorded n times. An n^{th}-time-recorded signal value with respect to the chemiluminescence is marked as RLUn.
(3) Any two signal values are selected from the signal values with respect to the chemiluminescence recorded in the n times and are marked as RLUm and RLUk, respectively. A growth rate from RLUm to RLUk is marked as A.
(4) A standard curve is made based on a series of known concentrations of standard substances containing the target molecules to be detected as well as a growth rate A' from RLUm' to RLUk' read with respect to any two reactions of the standard substances happened in step (2) and step (3). The concentrations of the standard substances are lower than a concentration at which the HOOK effect occurs.
(5) If the growth rate A is greater than a maximum value of the standard curve, then the sample to be detected is diluted and measured again.

t, n, m, and k are natural numbers greater than 0, and k<m≤n≤t, n≥2.

In some embodiments of the present disclosure, the growth rate A=(RLUm/RLUk-1)×100%.

In other embodiments of the present disclosure, n is greater than 2. For example, n is 3, 4 or 5 etc. In the present disclosure, when n is greater than 2, the kit exhibits relatively high detection sensitivity and strong anti-HD-HOOK-effect capability.

In some preferred embodiments of the present disclosure, the standard substance is used as a positive control.

In some embodiments of the present disclosure, in step (1), the chemiluminescent immunoreaction is a homogeneous chemiluminescent immunoreaction.

In some preferred embodiments of the present disclosure, in step (1), the reagents required for the chemiluminescent immunoreaction include an acceptor reagent and a donor reagent. The donor reagent includes a donor which is capable of generating singlet oxygen in an excited state. The acceptor reagent includes an acceptor which is capable of reacting with singlet oxygen to generate a detectable chemiluminescence signal value.

In some embodiments of the present disclosure, the acceptor is polymer particles filled with a light-emitting compound and a lanthanide compound.

In some preferred embodiments of the present disclosure, the light-emitting compound is selected from olefin compounds, preferably selected from thioxene, dibutanedione compounds, dioxene, enol ether, enamine, 9-alkylene xanthane, 9-alkylene-N-9,10 dihydroacridine, aryl ether alkene, aryl imidazole, lucigenin, and their derivatives, more preferably selected from thioxene and its derivatives.

In other preferred embodiments of the present disclosure, the lanthanide compound is a europium complex.

In some embodiments of the present disclosure, the acceptor includes an olefin compound and a metal chelate which are in a non-particle form and are soluble in a water-bearing medium.

In some specific embodiments of the present disclosure, the acceptor binds directly or indirectly to a first binding conjugate specific to the target molecules to be detected.

In some embodiments of the present disclosure, the donor is polymer particles filled with a photosensitive compound which is capable of generating singlet oxygen when excited by a red laser beam.

In other embodiments of the present disclosure, the photosensitive compound is one selected from methylene blue, rose red, porphyrin, and phthalocyanine.

In some embodiments of the present disclosure, the donor binds directly or indirectly to a label.

In some preferred embodiments of the present disclosure, in step (1), the reagents required for the chemiluminescent immunoreaction further include a second binding conjugate specific to the target molecules to be detected. Preferably, the second binding conjugate specific to the target molecules to be detected binds directly or indirectly to a binding conjugate specific to the label.

In some embodiments of the present disclosure, in step (1), the sample to be detected containing the target molecules to be detected is first mixed with the acceptor reagent and the second binding conjugate reagent specific to the target molecules to be detected, and then mixed with the donor reagent.

In some specific embodiments of the present disclosure, in step (2), the mixture to be detected is excited to undergo the chemiluminescence by means of energy and/or an active compound. Preferably, the mixture to be detected is irradiated with red excitation light of 600-700 nm so as to be excited to undergo the chemiluminescence.

In some other specific embodiments of the present disclosure, in step (2), a wavelength that is detected for recording the signal values with respect to the chemiluminescence is 520-620 nm.

In some embodiments of the present disclosure, the target molecules to be detected are an antigen or an antibody. The antigen is an immunogenic substance, and the antibody is an immunoglobulin which is produced by the body capable of recognizing a unique foreign substance.

In some specific embodiments of the present disclosure, a method of using the kit specifically includes the following steps.
(a1) A sample to be detected suspected of containing an antigen (or antibody) to be detected is mixed with an acceptor reagent and then subjected to a first incubation. A mixed solution obtained from the first incubation is then mixed with a donor reagent and subjected to a second incubation to form a mixture to be detected.
(a2) The mixture to be detected is excited successively t times with red excitation light of 600-700 nm to undergo chemiluminescence, and signal values with respect to the chemiluminescence are recorded n times. A wavelength detected is 520-620 nm. An n^{th}-time-recorded signal value with respect to the chemiluminescence is marked as RLUn.
(a3) Any two signal values are selected from the signal values with respect to the chemiluminescence recorded in the n times and are marked as RLUm and RLUk, respectively. A growth rate from RLUm to RLUk is marked as A. The growth rate A=(RLUm/RLUk-1)×100%.
(a4) A standard curve is made based on a series of known concentrations of standard substances containing the target molecules to be detected as well as a growth rate A' from RLUm' to RLUk' read with respect to any two reactions of the standard substances happened in step (2) and step (3). The concentrations of the standard substances are lower than a concentration at which the HOOK effect occurs.
(a5) If the growth rate A is greater than a maximum value of the standard curve, then the sample to be detected is diluted and measured again.

t, n, m, and k are natural numbers greater than 0, and k<m≤n≤t, n≥2.

The present disclosure, according to a fourth aspect thereof, relates to use of the method according to the first aspect of the present disclosure, the system according to the second aspect of the present disclosure, or the kit according to the third aspect of the present disclosure, in AFP detection.

Here, it shall be specifically noted that the above method is not a method for diagnosis of diseases. The method is used in a double-antibody sandwich immunoassay or used in a detection using a double-antigen sandwich immunoassay, to broaden a detection range and indicate an HD-HOOK sample or a sample that is beyond the detection range by way of selecting two signal values from signal values that are read in multiple times.

Preferably, the antigen is an immunogenic substance such as proteins and polypeptides. Typical antigens include (but are not limited to): cytokines, tumor makers, metalloproteins, cardiovascular diseaseglycuresis related proteins, etc.

The antibody refers to an immunoglobulin which is produced by the body capable of recognizing a unique foreign substance.

In the present disclosure, the antigen or antibody may be selected from alpha-fetoprotein (AFP), hepatitis B virus surface antibody (HBsAb), human chorionic gonadotropin and β subunit (HCG+β), hepatitis B surface antigen (HBsAg), cancer antigen 125 (CA125), C-peptide (CP), ferritin (Ferr), anti-HCV, etc.

Samples that can be detected by the method of the present disclosure are not particularly limited, and may be any sample containing a target antigen (or antibody) to be detected. Typical examples include serum samples, urine samples, saliva samples, etc. A preferred sample used in the present disclosure is a serum sample.

Preferably, the label and the binding conjugate specific to the label may specifically bind to each other.

More preferably, the label is biotin, and the binding conjugate specific to the label is streptavidin.

Preferably, the acceptor is polymer particles filled with a light-emitting compound and a lanthanide compound. The light-emitting compound may be a derivative of dioxene or thioxene etc., and the lanthanide compound can be Eu(TTA)3/TOPO or Eu(TTA)3/Phen. Such particles are commercially available. A surface functional group of the acceptor may be any groups that can bind to a protein, which are for example, various known functional groups such as carboxyl group, aldehyde group, amidogen, epoxy ethyl, or haloalkyl group that can bind to a protein.

Preferably, the donor is polymer particles filled with a photosensitive compound. The polymer particles are capable of generating singlet oxygen ions when excited by a red laser beam. When the polymer particles are close enough to the acceptor, the singlet oxygen ions travel to an acceptor and react with the light-emitting compound in the acceptor to produce ultraviolet light. The ultraviolet light further excites the lanthanide compound to produce photons of a certain wavelength. The photosensitive compound may be a phthalocyanine dye or the like. The particles are also commercially available.

Within a detection range, the concentration of the target antigen to be detected is reflected by the number of double-antibody sandwich complexes and is in direct proportion to the number of photons. When the concentration of the target antigen to be detected is too high, some of the antigens to be detected bind to a single antibody, as a consequence of which less double-antibody sandwich complexes are formed. This leads to a low light signal which cannot accurately reflect the real concentration of the target antigen to be detected.

Similarly, within the detection range, the concentration of the target antibody to be detected is reflected by the number of the double-antigen sandwich complexes and is in direct proportion to the number of photons. When the concentration of the target antibody to be detected is too high, some of the antibodies to be detected bind to a single antigen, as a consequence of which less double-antigen sandwich complexes are formed. This leads to a low light signal which cannot accurately reflect the real concentration of the target antibody to be detected.

The method of the present disclosure broadens the detection range and indicates an HD-HOOK sample or a sample that is beyond the detection range by way of selecting two signal values from signal values that are read in multiple times. A difference between the two signal values read in the two times is determined from the following three aspects.

First, when the signal value is read a first time, the acceptor releases singlet oxygen ions in response to the irradiation of the red laser beam (600-700 nm). Some of the singlet oxygen ions travel to the acceptor, and emit, after a series of chemical reactions, high energy light of 520-620 nm; and some other singlet oxygen ions react with the target antigen (or antibody) to be detected that is not bound by the antibody (or antigen). This reduces the concentration of the target antigen (or antibody) to be detected. For a low-concentration sample, after the concentration of the target antigen (or antibody) to be detected decreases, less double-antibody sandwich complexes are formed, and therefore the signal value read a second time is smaller. For a high-concentration sample, after the concentration of the target antigen (or antibody) to be detected is reduced, more double-antibody sandwich complexes are formed, and therefore the signal value read the second time is larger.

Second, for a low-concentration sample, when the signal value is read the first time, the donor releases singlet oxygen ions when irradiated by the red laser beam (600-700 nm) and some energy thereof is consumed. That is why the signal value read the second time is smaller.

Third, for an HD-HOOK-effect sample, when the signal value is read the first time, the antigen-antibody reaction does not reach equilibrium, and during an interval between the two times of reading, the reaction keeps proceeding in a forward direction. That is why the signal value read the second time is larger.

To summarize, in the present disclosure, the signal value is read the first time when the reaction does not reach equilibrium. At this time, the donor releases singlet oxygen ions when irradiated by the red laser beam. Some of the singlet oxygen ions travel to the acceptor; some other singlet oxygen ions can react with the target antigen (or antibody) to be detected that is not bound; and part of the target antigen (or antibody) to be detected is consumed. In this way, the equilibrium of the reaction is shifted in a reverse direction. On the other hand, after the donor is excited once, it is somehow consumed, and therefore when the signal value is read the second time, the signal value of the sample to be detected at a low concentration of the target antigen (or antibody) is smaller. For a high-concentration sample, when the signal value is read the first time, the binding between the double-antibody sandwich complexes and the donor is far from equilibrium, and when the signal value is read the second time, the reaction shifts in the forward direction, and therefore the signal value is larger.

### III. Emobodiments

In order to make the present disclosure easier to understand, the present disclosure will be further described in detail below in conjunction with embodiments. These embodiments are only illustrative and are not intended to limit the scope of application of the present disclosure. Unless otherwise specified, raw materials or components used in the present disclosure are commercially available or can be prepared through conventional methods.

### Example 1: Detection of alpha-fetoprotein (AFP) in a sample by a conventional method and the method of the present disclosure respectively

Alpha-fetoprotein (AFP) detection kit (chemiluminescence) manufactured by Shanghai Beyond Biotech Co., Ltd was used in this Example to measure a concentration of alpha-fetoprotein in a sample.

Gradient dilution was performed on a high-concentration alpha-fetoprotein antigen. Signal values of samples at different concentrations of alpha-fetoprotein were detected by a conventional method and by the method of the present disclosure, respectively.

Detection by the conventional method included the following steps.

A sample to be detected at a known concentration (a known standard substance), a reagent 1 (which was an acceptor solution having bound to a mouse monoclonal antibody), and a reagent 2 (a mouse monoclonal antibody solution having bound to biotin) were mixed, and then incubated at 37°C for 15 min, followed by addition of a reagent 3 (a donor solution having bound to streptavidin) to obtain a mixture. The mixture was incubated at 37°C for 10 min to obtain a reaction solution. The reaction solution was irradiated with a laser beam. A photon counter was used to read a signal value which was marked as RLU. Results are shown in Table 1.

Detection by the method of the present disclosure in which signal values were read multiple times included the following steps.

A sample to be detected at a known concentration (a known standard substance), a reagent 1 (which was an acceptor solution having bound to a mouse monoclonal antibody), and a reagent 2 (a mouse monoclonal antibody solution having bound to biotin) were mixed, and then incubated at 37°C for 15 min, followed by addition of a reagent 3 (a donor solution having bound to streptavidin) to obtain a mixture. The mixture was incubated at 37°C for 1 min, and a signal value was read and marked as RLU1(1min). The mixture was then incubated at 37°C, and when a total time of the two incubation amounted to 5 min, a signal value was read again and marked as RLU2(5min). The mixture was again incubated at 37°C, and when a total time of the three incubation amounted 10 min, a signal value was read and marked as RLU3(10min). The mixture was then incubated at 37°C, and when a total time of the four incubation amounted 20 min, a signal value was read and marked as RLU4(20min). Two of the signal values were selected, and a growth rate A between the two selected signal values was calculated based on equation A=(RLUm/RLUk-1)×100%, k < m≤n. Results are shown in Table 1.

**Table 1**

| AFP concentration | Time at which signal value was read (min) | | | | Growth rate A between two signal values | | | |
|---|---|---|---|---|---|---|---|---|
| ng/mL | RLU1 (1min) | RLU2 (5min) | RLU3 (10min) | RLU4 (20min) | (RLU2/RLU1-1) × 100% | (RLU3/RLU1-1) × 100% | (RLU4/RLU1-1) × 100% | (RLU4/RLU2-1) × 100% |
| 50 | 2219 | 1560 | 1217 | 1057 | -29.69% | -45.17% | -52.37% | -32.25% |
| 200 | 6014 | 4453 | 3420 | 2945 | -25.95% | -43.14% | -51.03% | -33.86% |
| 800 | 26273 | 25056 | 20516 | 17906 | -4.63% | -21.91% | -31.85% | -28.53% |
| 3200 | 116831 | 155320 | 144980 | 135291 | 32.94% | 24.09% | 15.80% | -12.90% |
| 12800 | 391561 | 581432 | 579317 | 567994 | 48.49% | 47.95% | 45.06% | -2.31% |
| 51200 | 688438 | 1079654 | 1117775 | 1160253 | 56.83% | 62.36% | 68.53% | 7.47% |
| 204800 | 474318 | 773523 | 819849 | 832642 | 63.08% | 72.85% | 75.55% | 7.64% |
| 819200 | 142500 | 238776 | 253665 | 260325 | 67.56% | 78.01% | 82.68% | 9.02% |
| 3276800 | 24940 | 42648 | 45614 | 46874 | 71.00% | 82.89% | 87.95% | 9.91% |

As can be seen from Table 1, as the concentration increases from 50 ng/ml to 51,200 ng/ml, the signal value increases with the increase of the concentration. As the concentration continues increasing, the signal value decreases with the increase of the concentration of alpha-fetoprotein. That is to say, when the concentration is higher than 51,200 ng/ml, the HD-HOOK effect occurs. In a conventional detection, a sample at an antigen concentration higher than such a detection range would be detected as having a relatively low antigen concentration (all concentrations detected would be lower than 51,200 ng/ml).

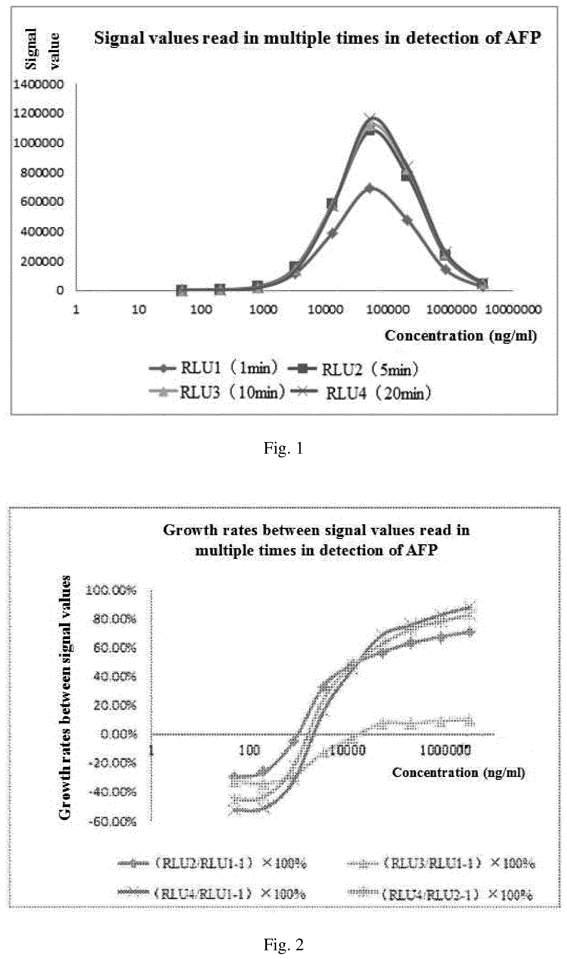

The method of the present disclosure broadens a detection range and indicates an HD-HOOK sample or a sample that is beyond the detection range by way of selecting two signal values from signal values that are read in multiple times. After detection of a series of samples at known concentrations, signal values are obtained. A standard curve of the concentrations and corresponding signal values and a standard curve of the concentrations and the growth rates A are drawn respectively (as shown in Fig. 1 and Fig. 2, respectively). RLU2(5min), as well as RLU4(20min) and RLU1(1min) of the sample to be detected that are read in two of the multiple times are selected. A growth rate A is calculated based on A=(RLU4/RLU1-1)×100%, and is used as one of indexes for judging a concentration range of a sample. As can be seen from Table 1 and Fig. 1, the signal value increases continuously with the increase of the concentration till the concentration reaches 51,200 ng/ml (defined as a rising section) and then begins to decrease with the increase of the concentration (defined as a dropping section), while the growth rate A keeps increasing with the increase of the concentration (Fig. 2). The method of the present disclosure is used to detect and obtain RLU1(1min), RLU2(5min), RLU3(10min), RLU4(20min), and A of the sample to be detected.

A standard curve of RLU2(5min) and a standard curve of A, within a rising section (such as 0-1,000 ng/ml), are drawn. A value of the growth rate A of the sample to be detected is compared with the standard curve of A first. If A<A(1000 ng/ml), then RLU2(5min) of the sample to be detected is substituted into the standard curve to calculate an exact concentration. If A>A(1000 ng/ml), it means that the concentration of the sample to be detected exceeds the range of the standard curve and needs to be diluted for detection.

### Example 2: Detection of human chorionic gonadotropin and β subunit (HCG+β) in a sample by a conventional method and the method of the present disclosure respectively

Human chorionic gonadotropin and β subunit (HCG+β) detection kit (chemiluminescence) manufactured by Shanghai Beyond Biotech Co., Ltd was used to measure a concentration of HCG+β in a sample.

Gradient dilution was performed on a high-concentration human chorionic gonadotropin and β subunit antigen. Signal values of samples at different concentrations of human chorionic gonadotropin and β subunit were detected by a conventional method and by the method of the present disclosure, respectively.

Detection by the conventional method: A sample to be detected at a known concentration, a reagent 1 (which was an acceptor solution having bound to a mouse monoclonal antibody), and a reagent 2 (a mouse monoclonal antibody solution having bound to biotin) were mixed, and then incubated at 37°C for 15 min, followed by addition of a reagent 3 (a donor solution having bound to streptavidin) to obtain a mixture. The mixture was incubated at 37°C for 10 min. A photon counter was used to read a signal value which was marked as RLU. Results are shown in Table 2.

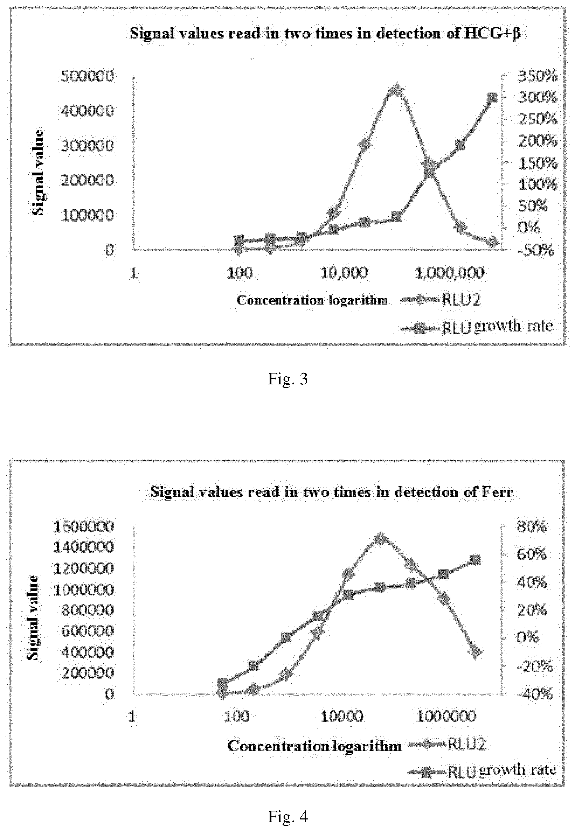

Detection by the method of the present disclosure in which signal values were read two times: A sample to be detected at a known concentration, a reagent 1 (which was a light-emitting antibody, namely light-emitting particles coated with a mouse monoclonal antibody), and a reagent 2 (a biotin-labeled antibody, namely a mouse monoclonal antibody labeled with biotin) were incubated at 37°C for 15 min, followed by addition of a general-purpose LiCA solution (light-sensitive particles labeled with streptavidin) to obtain a mixture. The mixture was incubated at 37°C for 3 min, and a signal value was read and marked as RLU1. The mixture was then incubated at 37°C for 7 min, and a signal value was read and marked as RLU2. A growth rate of the signal value read in the second time was calculated based on equation A= (RLU2/RLU1-1)×100%. Results are shown in Table 2 and Fig. 3.

**Table 2**

| Serial number of samples | Concentration (mIU/ml) | Results of detection by the conventional method | Results of detection by the method of the present disclosure | | |
|---|---|---|---|---|---|
| | | RLU | RLU1 | RLU2 | Growth rate A |
| 1# | 100 | 4783 | 6073 | 4217 | -31% |
| 2# | 400 | 16153 | 22297 | 15630 | -30% |
| 3# | 1,600 | 68730 | 85405 | 66818 | -22% |
| 4# | 6,400 | 282534 | 283179 | 263684 | -7% |
| 5# | 25,600 | 844830 | 663207 | 741788 | 12% |
| 6# | 102,400 | 1162159 | 855280 | 1044928 | 22% |
| 7# | 409,600 | 395703 | 272396 | 378001 | 39% |
| 8# | 1,638,400 | 97248 | 56037 | 88323 | 58% |
| 9# | 6,553,600 | 23462 | 13174 | 21321 | 62% |

As can be seen from Table 2, as the concentration increases from 100 mIU/ml to 102,400 mIU/ml, the signal value increases with the increase of the concentration. As the concentration continues increasing, the signal value decreases with the increase of the concentration of human chorionic gonadotropin and β subunit. That is to say, when the concentration is higher than 102,400 mIU/ml (this concentration is defined as an HD-HOOK-effect inflection point, and its growth rate is defined as Ao), the HD-HOOK effect occurs. In a conventional detection, a sample at an antigen concentration higher than such a detection range would be detected as having a relatively low antigen concentration (all concentrations detected would be lower than 102,400 mIU/ml).

The method of the present disclosure broadens a detection range and indicates an HD-HOOK sample or a sample that is beyond the detection range by means of signal values read in two times. Each sample to be detected is successively detected to obtain signal values RLU1, RLU2. The growth rate of the signal value RUL read in the second time is taken as one of indexes for judging the concentration range of the sample. As can be seen from Table 2 and Fig. 3, the signal value increases continuously with the increase of the concentration till the concentration reaches 51,200 mIU/ml (defined as a rising section) and then begins to decrease with the increase of the concentration (defined as a dropping section), while the growth rate A keeps increasing with the increase of the concentration. The method of the present disclosure is used to detect and obtain RLU1, RLU2, and A of the sample to be detected.

A standard curve of RLU2 and a standard curve of A, within a rising section (such as 0-1,0000 mIU/ml), are drawn. A value of the growth rate A of the sample to be detected is compared with the standard curve of A first. If A<A_{(1,0000mIU/ml)}, then RLU2 of the sample to be detected is substituted into the standard curve to calculate an exact concentration. If A>A_{(1,0000 mIU/ml)}, it means that the concentration of the sample to be detected exceeds the range of the standard curve and needs to be diluted for detection.

### Example 3: Detection of ferritin (Ferr) in a sample by a conventional method and the method of the present disclosure respectively

Ferritin (Ferr) detection kit (chemiluminescence) manufactured by Shanghai Beyond Biotech Co., Ltd was used to measure a concentration of ferritin in a sample.

Gradient dilution was performed on a high-concentration ferritin antigen. Signal values of samples at different concentrations of ferritin were detected by a conventional method and by the method of the present disclosure, respectively.

Detection by the conventional method: A sample to be detected at a known concentration, a reagent 1 (which was a light-emitting antibody, namely light-emitting particles coated with a mouse monoclonal antibody), and a reagent 2 (which was a biotin-labeled antibody, namely a mouse monoclonal antibody labeled with biotin) were added into a reaction vessel, and then incubated at 37°C for 15 min, followed by addition of a general-purpose LiCA solution (light-sensitive particles labeled with streptavidin) to obtain a mixture. The mixture was incubated at 37°C for 10 min. A photon counter was used to read a signal value which was marked as RLU. Results are shown in Table 3.

Detection by the method of the present disclosure in which signal values were read two times: A sample to be detected at a known concentration, a reagent 1 (which was a light-emitting antibody, namely light-emitting particles coated with a mouse monoclonal antibody), and a reagent 2 (which was a biotin-labeled antibody, namely a mouse monoclonal antibody labeled with biotin) were mixed, and then incubated at 37°C for 15 min, followed by addition of a general-purpose LiCA solution (light-sensitive particles labeled with streptavidin) to obtain a mixture. The mixture was incubated at 37°C for 3 min, and a signal value was read and marked as RLU1. The mixture was then incubated at 37°C for 7 min, and a signal value was read and marked as RLU2. A growth rate of the signal value read in the second time was calculated based on equation A=(RLU2/RLU1-1)×100%. Results are shown in Table 3 and Fig. 4.

**Table 3**

| Serial number of samples | Concentration (ng/ml) | Results of detection by the conventional method | Results of detection by the method of the present disclosure | | |
|---|---|---|---|---|---|
| | | RLU | RLU1 | RLU2 | Growth rate A |
| 1# | 50 | 29374 | 36982 | 29226 | -21% |
| 2# | 200 | 130237 | 139538 | 118990 | -15% |
| 3# | 800 | 514946 | 470907 | 467257 | -1% |
| 4# | 3200 | 1631905 | 1219373 | 1437994 | 18% |
| 5# | 12800 | 2994937 | 2280569 | 2821545 | 24% |
| 6# | 51200 | 3402607 | 2578903 | 3163238 | 23% |
| 7# | 204800 | 3272694 | 2314036 | 2907751 | 26% |
| 8# | 819200 | 2437951 | 1645331 | 2165755 | 32% |
| 9# | 3276800 | 1002072 | 633495 | 948425 | 50% |

As can be seen from Table 3, as the concentration increases from 50 ng/ml to 51,200 ng/ml, the signal value increases with the increase of the concentration. As the concentration continues increasing, the signal value decreases with the increase of the concentration of ferritin. That is to say, when the concentration is higher than 51,200 ng/ml (this concentration is defined as an HD-HOOK-effect inflection point, and its growth rate is defined as A₀), the HD-HOOK effect occurs. In a conventional detection, a sample at an antigen concentration higher than such a detection range would be detected as having a relatively low antigen concentration (all concentrations detected would be lower than 51,200 ng/ml).

The method of the present disclosure broadens a detection range and indicates an HD-HOOK sample or a sample that is beyond the detection range by means of signal values read in two times. Each sample to be detected is successively detected to obtain signal values RLU1, RLU2. The growth rate of the signal value RUL read in the second time is taken as one of indexes for judging the concentration range of the sample. As can be seen from Table 3 and Fig. 4, the signal value increases continuously with the increase of the concentration till the concentration reaches 51,200 ng/ml (defined as a rising section) and then begins to decrease with the increase of the concentration (defined as a dropping section), while the growth rate A keeps increasing with the increase of the concentration. The method of the present disclosure is used to detect and obtain RLU1, RLU2, and A of the sample to be detected.

A standard curve of RLU2 and a standard curve of A, within a rising section (such as 0-1,000 ng/ml), are drawn. A value of the growth rate A of the sample to be detected is compared with the standard curve of A first. If A<A_{(1,000ng/ml)}, then RLU2 of the sample to be detected is substituted into the standard curve to calculate an exact concentration. If A>A_{(1,000 ng/ml)}, it means that the concentration of the sample to be detected exceeds the range of the standard curve and needs to be diluted for detection.

### Example 4: Detection of human immunodeficiency virus antibody (anti-HIV) in a sample by a conventional method and the method of the present disclosure respectively

Human immunodeficiency virus antibody (anti-HIV) detection kit (chemiluminescence) manufactured by Shanghai Beyond Biotech Co., Ltd was used to measure a concentration of human immunodeficiency virus antibody in a sample.

Gradient dilution was performed on a high-concentration human immunodeficiency virus antibody. Signal values of samples at different concentrations of human immunodeficiency virus antibody were detected by a conventional method and by the method of the present disclosure, respectively.

Detection by the conventional method: A sample to be detected at a known concentration, a reagent 1 (which was a light-emitting reagent, namely light-emitting particles coated with an HIV antigen), and a reagent 2 (which was a biotin reagent, namely an HIV antigen labeled with biotin) were added into a reaction vessel, and then incubated at 37°C for 15 min, followed by addition of a general-purpose LiCA solution (light-sensitive particles labeled with streptavidin) to obtain a mixture. The mixture was incubated at 37°C for 10 min. A photon counter was used to read a signal value which was marked as RLU. Results are shown in Table 4.

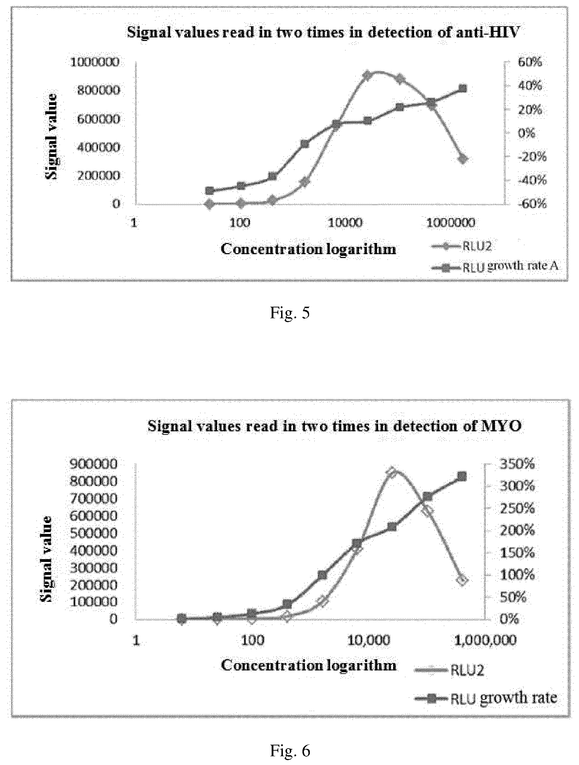

Detection by the method of the present disclosure in which signal values were read two times: A sample to be detected at a known concentration, a reagent 1 (which was a light-emitting reagent, namely light-emitting particles coated with an HIV antigen), and a reagent 2 (which was a biotin reagent, namely an HIV antigen labeled with biotin) were mixed, and then incubated at 37°C for 15 min, followed by addition of a general-purpose LiCA solution (light-sensitive particles labeled with streptavidin) to obtain a mixture. The mixture was incubated at 37°C for 3 min, and a signal value was read and marked as RLU1. The mixture was then incubated at 37°C for 7 min, and a signal value was read and marked as RLU2. A growth rate of the signal value read in the second time was calculated based on equation A=(RLU2/RLU1-1)×100%. Results are shown in Table 4 and Fig. 5.

**Table 4**

| Serial number of samples | Concentration (ng/ml) | Results of detection by the conventional method | Results of detection by the method of the present disclosure | | |
|---|---|---|---|---|---|
| | | RLU | RLU1 | RLU2 | Growth rate A |
| 1# | 25 | 3187 | 6172 | 3086 | -50% |
| 2# | 100 | 13310 | 21969 | 11467 | -48% |
| 3# | 400 | 66789 | 97480 | 61403 | -37% |
| 4# | 1600 | 403890 | 426498 | 380495 | -11% |
| 5# | 6400 | 1591893 | 1301691 | 1488670 | 14% |
| 6# | 25600 | 2683158 | 1921236 | 2331572 | 21% |
| 7# | 102400 | 2497961 | 1829868 | 2258869 | 23% |
| 8# | 409600 | 1854742 | 1305503 | 1712345 | 31% |
| 9# | 1638400 | 820651 | 539365 | 778920 | 44% |

As can be seen from Table 4, as the concentration increases from 25 ng/ml to 25,600 ng/ml, the signal value increases with the increase of the concentration. As the concentration continues increasing, the signal value decreases with the increase of the concentration of the human immunodeficiency virus antibody. That is to say, when the concentration is higher than 25,600 ng/ml (this concentration is defined as an HD-HOOK-effect inflection point, and its growth rate is defined as Ao), the HD-HOOK effect occurs. In a conventional detection, a sample at an antigen concentration higher than such a detection range would be detected as having a relatively low antigen concentration (all concentrations detected would be lower than 25,600 ng/ml).

The method of the present disclosure broadens a detection range and indicates an HD-HOOK sample or a sample that is beyond the detection range by means of signal values read in two times. Each sample to be detected is successively detected to obtain signal values RLU1 and RLU2. The growth rate of the signal value RUL read in the second time is taken as one of indexes for judging the concentration range of the sample. As can be seen from Table 4 and Fig. 5, the signal value increases continuously with the increase of the concentration till the concentration reaches 25,600 ng/ml (defined as a rising section) and then begins to decrease with the increase of the concentration (defined as a dropping section), while the growth rate A keeps increasing with the increase of the concentration. The method of the present disclosure is used to detect and obtain RLU1, RLU2, and A of the sample to be detected.

A standard curve of RLU2 and a standard curve of A, within a rising section (such as 0-1,000 ng/ml), are drawn. A value of the growth rate A of the sample to be detected is compared with the standard curve of A first. If A<A_{(1,000 ng/ml)}, then RLU2 of the sample to be detected is substituted into the standard curve to calculate an exact concentration. If A>A_{(1,000 ng/ml)}, it means that the concentration of the sample to be detected exceeds the range of the standard curve and needs to be diluted for detection.

### Example 5: Detection of myoglobin (MYO) in a sample by a conventional method and the method of the present disclosure respectively

Myoglobin (MYO) detection kit (chemiluminescence) manufactured by Shanghai Beyond Biotech Co., Ltd was used to measure a concentration of myoglobin in a sample.

Gradient dilution was performed on a high-concentration myoglobin antigen. Signal values of samples at different concentrations of myoglobin were detected by a conventional method and by the method of the present disclosure, respectively.

Detection by the conventional method: A sample to be detected at a known concentration, a reagent 1 (which was a light-emitting antibody, namely light-emitting particles coated with a mouse monoclonal antibody), and a reagent 2 (a biotin-labeled antibody, namely a mouse monoclonal antibody labeled with biotin) were added into a reaction vessel, and then incubated at 37°C for 15 min, followed by addition of a general-purpose LiCA solution (which was light-sensitive particles labeled with streptavidin) to obtain a mixture. The mixture was incubated at 37°C for 10 min. A photon counter was used to read a signal value which was marked as RLU. Results are shown in Table 5.

Detection by the method of the present disclosure in which signal values were read two times: A sample to be detected at a known concentration, a reagent 1 (which was a light-emitting antibody, namely light-emitting particles coated with a mouse monoclonal antibody), and a reagent 2 (a biotin-labeled antibody, namely a mouse monoclonal antibody labeled with biotin) were mixed, and then incubated at 37°C for 15 min, followed by addition of a general-purpose LiCA solution (which were light-sensitive particles labeled with streptavidin) to obtain a mixture. The mixture was incubated at 37°C for 3 min, and a signal value was read and marked as RLU1. The mixture was then incubated at 37°C for 7 min, and a signal value was read and marked as RLU2. A growth rate of the signal value read in the second time was calculated based on equation A=(RLU2/RLU1-1)×100%. Results are shown in Table 5 and Fig. 6.

**Table 5**

| Serial number of samples | Concentration (ng/ml) | Results of detection by the conventional method | Results of detection by the method of the present disclosure | | |
|---|---|---|---|---|---|
| | | RLU | RLU1 | RLU2 | Growth rate A |
| 1# | 6 | 4087 | 3001 | 3124 | 4% |
| 2# | 25 | 5904 | 4450 | 4679 | 5% |
| 3# | 100 | 13489 | 10081 | 11069 | 10% |
| 4# | 400 | 44629 | 35422 | 46248 | 31% |
| 5# | 1,600 | 167251 | 130712 | 261070 | 100% |
| 6# | 6,400 | 468674 | 374761 | 1025554 | 174% |
| 7# | 25,600 | 832315 | 677602 | 2060119 | 204% |
| 8# | 102,400 | 539604 | 417102 | 1574654 | 278% |
| 9# | 409,600 | 179718 | 134668 | 549601 | 308% |

As can be seen from Table 5, as the concentration increases from 6 ng/ml to 25,600 ng/ml, the signal value increases with the increase of the concentration. As the concentration continues increasing, the signal value decreases with the increase of the concentration of myoglobin. That is to say, when the concentration is higher than 25,600 ng/ml (this concentration is defined as an HD-HOOK-effect inflection point, and its growth rate is defined as A₀), the HD-HOOK effect occurs. In a conventional detection, a sample at an antigen concentration higher than such a detection range would be detected as having a relatively low antigen concentration (all concentrations detected would be lower than 25,600 ng/ml).

The method of the present disclosure broadens a detection range and indicates an HD-HOOK sample or a sample that is beyond the detection range by means of signal values read in two times. Each sample to be detected is successively detected to obtain signal values RLU1, RLU2. The growth rate of the signal value RUL read in the second time is taken as one of indexes for judging the concentration range of the sample. As can be seen from Table 5 and Fig. 6, the signal value increases continuously with the increase of the concentration till the concentration reaches 25,600 ng/ml (defined as a rising section) and then begins to decrease with the increase of the concentration (defined as a dropping section), while the growth rate A keeps increasing with the increase of the concentration. The method of the present disclosure is used to detect and obtain RLU1, RLU2, and A of the sample to be detected.

A standard curve of RLU2 and a standard curve of A, within a rising section (such as 0-1,000 ng/ml), are drawn. A value of the growth rate A of the sample to be detected is compared with the standard curve of A first. If A<A_{(1,000 ng/ml)}, then RLU2 of the sample to be detected is substituted into the standard curve to calculate an exact concentration. If A>A_{(1,000 ng/ml)}, it means that the concentration of the sample to be detected exceeds the range of the standard curve and needs to be diluted for detection.

### Example 6: Detection of N-terminal atrial natriuretic peptide (NT-proBNP) in a sample by a conventional method and the method of the present disclosure respectively

N-terminal atrial natriuretic peptide (NT-proBNP) detection kit (chemiluminescence) manufactured by Shanghai Beyond Biotech Co., Ltd was used to measure a concentration of N-terminal atrial natriuretic peptide in a sample.

Gradient dilution was performed on a high-concentration N-terminal atrial natriuretic peptide antigen. Signal values of samples at different concentrations of N-terminal atrial natriuretic peptide were detected by a conventional method and by the method of the present disclosure, respectively.

Detection by the conventional method: A sample to be detected at a known concentration, a reagent 1 (which was a light-emitting antibody, namely light-emitting particles coated with a mouse monoclonal antibody), and a reagent 2 (a biotin-labeled antibody, namely a mouse monoclonal antibody labeled with biotin) were added into a reaction vessel, and then incubated at 37°C for 15 min, followed by addition of a general-purpose LiCA solution (light-sensitive particles labeled with streptavidin) to obtain a mixture. The mixture was incubated at 37°C for 10 min. A photon counter was used to read a signal value which was marked as RLU. Results are shown in Table 6.

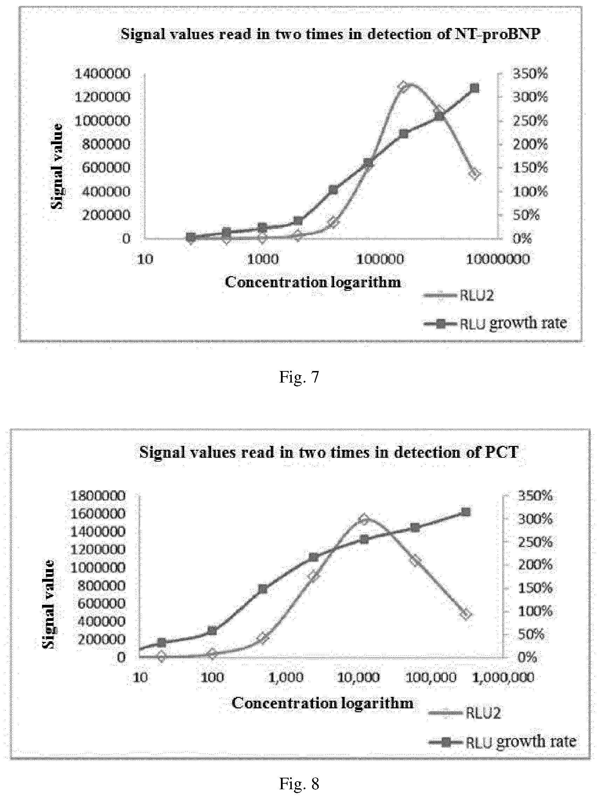

Detection by the method of the present disclosure in which signal values were read two times: A sample to be detected at a known concentration, a reagent 1 (which was a light-emitting antibody, namely light-emitting particles coated with a mouse monoclonal antibody), and a reagent 2 (which was a biotin-labeled antibody, namely a mouse monoclonal antibody labeled with biotin) were mixed, and then incubated at 37°C for 15 min, followed by addition of a general-purpose LiCA solution (light-sensitive particles labeled with streptavidin) to obtain a mixture. The mixture was incubated at 37°C for 3 min, and a signal value was read and marked as RLU1. The mixture was then incubated at 37°C for 7 min, and a signal value was read and marked as RLU2. A growth rate of the signal value read in the second time was calculated based on equation A=(RLU2/RLU1-1)×100%. Results are shown in Table 6 and Fig. 7.

**Table 6**

| Serial number of samples | Concentration (pg/ml) | Results of detection by the conventional method | Results of detection by the method of the present disclosure | | |
|---|---|---|---|---|---|
| | | RLU | RLU1 | RLU2 | Growth rate A |
| 1# | 62.5 | 6022 | 4751 | 4847 | 2% |
| 2# | 250 | 8162 | 6507 | 7053 | 8% |
| 3# | 1000 | 19752 | 16182 | 19168 | 18% |
| 4# | 4000 | 71440 | 50520 | 71309 | 41% |
| 5# | 16000 | 211974 | 163755 | 337723 | 106% |
| 6# | 64000 | 750435 | 587531 | 1555828 | 165% |
| 7# | 256000 | 1327403 | 1011360 | 3203183 | 217% |
| 8# | 1024000 | 923568 | 735261 | 2686846 | 265% |
| 9# | 4096000 | 424535 | 322636 | 1343679 | 316% |

As can be seen from Table 6, as the concentration increases from 62.5 pg/ml to 256,000 pg/ml, the signal value increases with the increase of the concentration. As the concentration continues increasing, the signal value decreases with the increase of the concentration of N-terminal atrial natriuretic peptide. That is to say, when the concentration is higher than 256,000 pg/ml (this concentration is defined as an HD-HOOK-effect inflection point, and its growth rate is defined as Ao), the HD-HOOK effect occurs. In a conventional detection, a sample at an antigen concentration higher than such a detection range would be detected as having a relatively low antigen concentration (all concentrations detected would be lower than 256,000 pg/ml).

The method of the present disclosure broadens a detection range and indicates an HD-HOOK sample or a sample that is beyond the detection range by means of signal values read in two times. Each sample to be detected is successively detected to obtain signal values RLU1, RLU2. The growth rate of the signal value RUL read in the second time is taken as one of indexes for judging the concentration range of the sample. As can be seen from Table 6 and Fig. 7, the signal value increases continuously with the increase of the concentration till the concentration reaches 256,000 pg/ml (defined as a rising section) and then begins to decrease with the increase of the concentration (defined as a dropping section), while the growth rate A keeps increasing with the increase of the concentration. The method of the present disclosure is used to detect and obtain RLU1, RLU2, and A of the sample to be detected.

A standard curve of RLU2 and a standard curve of A, within a rising section (such as 0-35,000 pg/ml), are drawn. A value of the growth rate A of the sample to be detected is compared with the standard curve of A first. If A<A_{(35,000 pg/ml)}, then RLU2 of the sample to be detected is substituted into the standard curve to calculate an exact concentration. If A>A_{(35,000 pg/ml)}, it means that the concentration of the sample to be detected exceeds the range of the standard curve and needs to be diluted for detection.

### Example 7: Detection of procalcitonin (PCT) in a sample by a conventional method and the method of the present disclosure respectively

Procalcitonin (PCT) detection kit (chemiluminescence) manufactured by Shanghai Beyond Biotech Co., Ltd was used to measure a concentration of procalcitonin in a sample.

Gradient dilution was performed on a high-concentration procalcitonin antigen. Signal values of samples at different concentrations of procalcitonin were detected by a conventional method and by the method of the present disclosure, respectively.

Detection by the conventional method: A sample to be detected at a known concentration, a reagent 1 (which was a light-emitting antibody, namely light-emitting particles coated with a mouse monoclonal antibody), and a reagent 2 (which was a biotin-labeled antibody, namely a mouse monoclonal antibody labeled with biotin) were added into a reaction vessel, and then incubated at 37°C for 15 min, followed by addition of a general-purpose LiCA solution(light-sensitive particles labeled with streptavidin) to obtain a mixture. The mixture was incubated at 37°C for 10 min. A photon counter was used to read a signal value which was marked as RLU. Results are shown in Table 7.

Detection by the method of the present disclosure in which signal values were read two times: A sample to be detected at a known concentration, a reagent 1 (which was a light-emitting antibody, namely light-emitting particles coated with a mouse monoclonal antibody), and a reagent 2 (which was a biotin-labeled antibody, namely a mouse monoclonal antibody labeled with biotin) were mixed, and then incubated at 37°C for 15 min, followed by addition of a general-purpose LiCA solution (light-sensitive particles labeled with streptavidin) to obtain a mixture. The mixture was incubated at 37°C for 3 min, and a signal value was read and marked as RLU1. The mixture was then incubated at 37°C for 7 min, and a signal value was read and marked as RLU2. A growth rate of the signal value read in the second time was calculated based on equation A=(RLU2/RLU1-1)×100%. Results are shown in Table 7 and Fig. 8.

**Table 7**

| Serial number of samples | Concentration (ng/ml) | Results of detection by the conventional method | Results of detection by the method of the present disclosure | | |
|---|---|---|---|---|---|
| | | RLU | RLU1 | RLU2 | Growth rate A |
| 1# | 1 | 6494 | 5677 | 6306 | 11% |
| 2# | 4 | 9300 | 8700 | 9265 | 6% |
| 3# | 20 | 23184 | 19058 | 25090 | 32% |
| 4# | 100 | 70813 | 65143 | 103267 | 59% |
| 5# | 500 | 252498 | 218813 | 537843 | 146% |
| 6# | 2,500 | 785933 | 716506 | 2206900 | 208% |
| 7# | 12,500 | 1220448 | 1104767 | 3872725 | 251% |
| 8# | 62,500 | 814576 | 708063 | 2664094 | 276% |
| 9# | 312,500 | 364549 | 293546 | 1198940 | 308% |

As can be seen from Table 7, as the concentration increases from 1 ng/ml to 12,500 ng/ml, the signal value increases with the increase of the concentration. As the concentration continues increasing, the signal value decreases with the increase of the concentration of procalcitonin. That is to say, when the concentration is higher than 12,500 ng/ml (this concentration is defined as an HD-HOOK-effect inflection point, and its growth rate is defined as A₀), the HD-HOOK effect occurs. In a conventional detection, a sample at an antigen concentration higher than such a detection range would be detected as having a relatively low antigen concentration (all concentrations detected would be lower than 12,500 ng/ml).

The method of the present disclosure broadens a detection range and indicates an HD-HOOK sample or a sample that is beyond the detection range by means of signal values read in two times. Each sample to be detected is successively detected to obtain signal values RLU1, RLU2. The growth rate of the signal value RUL read in the second time is taken as one of indexes for judging the concentration range of the sample. As can be seen from Table 7 and Fig. 8, the signal value increases continuously with the increase of the concentration till the concentration reaches 12,500 ng/ml (defined as a rising section) and then begins to decrease with the increase of the concentration (defined as a dropping section), while the growth rate A keeps increasing with the increase of the concentration. The method of the present disclosure is used to detect and obtain RLU1, RLU2, and A of the sample to be detected.

A standard curve of RLU2 and a standard curve of A, within a rising section (such as 0-100 ng/ml), are drawn. A value of the growth rate A of the sample to be detected is compared with the standard curve of A first. If A <A(_{100ng/ml}), then RLU2 of the sample to be detected is substituted into the standard curve to calculate an exact concentration. If A> A(_{100ng/ml}), it means that the concentration of the sample to be detected exceeds the range of the standard curve and needs to be diluted for detection.

### Example 8: Detection of troponin I (cTnI) in a sample by a conventional method and the method of the present disclosure respectively

Troponin I (cTnI) detection kit (chemiluminescence) manufactured by Shanghai Beyond Biotech Co., Ltd was used to measure a concentration of Troponin I in a sample.

Gradient dilution was performed on a high-concentration troponin I antigen. Signal values of samples at different concentrations of troponin I were detected by a conventional method and by the method of the present disclosure, respectively.

Detection by the conventional method: A sample to be detected at a known concentration, a reagent 1 (which was a light-emitting antibody, namely light-emitting particles coated with a mouse monoclonal antibody), and a reagent 2 (which was a biotin-labeled antibody, namely a mouse monoclonal antibody labeled with biotin) were added into a reaction vessel, and then incubated at 37°C for 15 min, followed by addition of a general-purpose LiCA solution(light-sensitive particles labeled with streptavidin) to obtain a mixture. The mixture was incubated at 37°C for 10 min. A photon counter was used to read a signal value which was marked as RLU. Results are shown in Table 8.

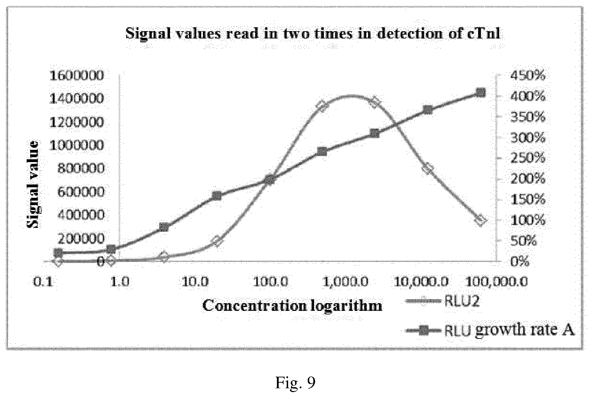

Detection by the method of the present disclosure in which signal values were read two times: A sample to be detected at a known concentration, a reagent 1 (which was a light-emitting antibody, namely light-emitting particles coated with a mouse monoclonal antibody), and a reagent 2 (a biotin-labeled antibody, namely a mouse monoclonal antibody labeled with biotin) were mixed, and then incubated at 37°C for 15 min, followed by addition of a general-purpose LiCA solution (which was light-sensitive particles labeled with streptavidin) to obtain a mixture. The mixture was incubated at 37°C for 3 min, and a signal value was read and marked as RLU1. The mixture was then incubated at 37°C for 7 min, and a signal value was read and marked as RLU2. A growth rate of the signal value read in the second time was calculated based on equation A=(RLU2/RLU1-1)×100%. Results are shown in Table 8 and Fig. 9.

**Table 8**

| Serial number of samples | Concentration (ng/ml) | Results of detection by the conventional method | Results of detection by the method of the present disclosure | | |
|---|---|---|---|---|---|
| | | RLU | RLU1 | RLU2 | Growth rate A |
| 1# | 0.2 | 7474 | 7047 | 8345 | 18% |
| 2# | 1 | 17361 | 15219 | 19797 | 30% |
| 3# | 4 | 55091 | 52344 | 95548 | 83% |
| 4# | 20 | 190395 | 174092 | 439092 | 152% |
| 5# | 100 | 635772 | 593405 | 1727512 | 191% |
| 6# | 500 | 961652 | 933015 | 3338403 | 258% |
| 7# | 2,500 | 865486 | 839713 | 3200562 | 281% |
| 8# | 12,500 | 455932 | 417650 | 1991517 | 377% |
| 9# | 62,500 | 101783 | 104668 | 549601 | 425% |

As can be seen from Table 8, as the concentration increases from 0.2 ng/ml to 500 ng/ml, the signal value increases with the increase of the concentration. As the concentration continues increasing, the signal value decreases with the increase of the concentration of troponin I. That is to say, when the concentration is higher than 500 ng/ml (this concentration is defined as an HD-HOOK-effect inflection point, and its growth rate is defined as A₀), the HD-HOOK effect occurs. In a conventional detection, a sample at an antigen concentration higher than such a detection range would be detected as having a relatively low antigen concentration (all concentrations detected would be lower than 500 ng/ml).

The method of the present disclosure broadens a detection range and indicates an HD-HOOK sample or a sample that is beyond the detection range by means of signal values read in two times. Each sample to be detected is successively detected to obtain signal values RLU1 and RLU2. The growth rate of the signal value RUL read in the second time is taken as one of indexes for judging the concentration range of the sample. As can be seen from Table 8 and Fig. 9, the signal value increases continuously with the increase of the concentration till the concentration reaches 500 ng/ml (defined as a rising section) and then begins to decrease with the increase of the concentration (defined as a dropping section), while the growth rate A keeps increasing with the increase of the concentration. The method of the present disclosure is used to detect and obtain RLU1, RLU2, and A of the sample to be detected.

A standard curve of RLU2 and a standard curve of A, within a rising section (such as 0-50 ng/ml), are drawn. A value of the growth rate A of the sample to be detected is compared with the standard curve of A first. If A<A(_{50ng/ml}), then RLU2 of the sample to be detected is substituted into the standard curve to calculate an exact concentration. If A>A(_{50 ng/ml}), it means that the concentration of the sample to be detected exceeds the range of the standard curve and needs to be diluted for detection.

It shall be noted that the above embodiments are indented only for explaining the present disclosure and do not constitute any limitation to the present disclosure. The present disclosure has been described with reference to representative embodiments, but it shall be appreciated that the words used therein are descriptive and explanatory rather than restrictive. Modifications may be made to the present disclosure within the scope of the claims of the present disclosure, and amendments may be made to the present disclosure without departing from the scope and spirit of the present disclosure. Although the present disclosure described therein relates to specific methods, materials and embodiments, it does not mean that the present disclosure is limited to these specific examples disclosed therein. On the contrary, the present disclosure can be extended to all other methods and applications with the same functions.

## Claims

1. A chemical luminescence immune analysis and measurement method, comprising the following steps:
(1) mixing and reacting a sample to be detected suspected of containing target molecules to be detected with reagents required for a chemiluminescent immunoreaction to form a mixture to be detected;
(2) exciting the mixture to be detected successively t times to cause the mixture to be detected to undergo chemiluminescence, and recording n times signal values with respect to the chemiluminescence, wherein an n^{th}-time-recorded signal value with respect to the chemiluminescence is marked as RLUn;
(3) selecting any two signal values from the signal values with respect to the chemiluminescence recorded in the n times, marking the selected two signal values as RLUm and RLUk, respectively, and marking a growth rate from RLUm to RLUk as A;
(4) making a standard curve based on a series of known concentrations of standard substances containing the target molecules to be detected as well as a growth rate A' from RLUm' to RLUk' read with respect to any two reactions of the standard substances happened in step (2) and step (3), wherein the concentrations of the standard substances are lower than a concentration at which a HOOK effect occurs; and
(5) diluting the sample to be detected and measuring the diluted sample to be detected again, if the growth rate A is greater than a maximum value of the standard curve,
wherein t, n, m, and k are natural numbers greater than 0, and k≤m≤n≤t, n≥2.

2. The method according to claim 1, wherein the growth rate A=(RLUm/RLUk-1 )×100%.

3. The method according to claim 1 or 2, wherein n is greater than 2.

4. The method according to any one of claims 1 to 3, wherein in step (1), the chemiluminescent immunoreaction is a homogeneous chemiluminescent immunoreaction.

5. The method according to any one of claims 1 to 4, wherein in step (1), the reagents required for the chemiluminescent immunoreaction comprises an acceptor reagent and a donor reagent, wherein:
the donor reagent comprises a donor which is capable of generating singlet oxygen in an excited state; and
the acceptor reagent comprises an acceptor which is capable of reacting with singlet oxygen to generate a detectable chemiluminescence signal value.

6. The method according to claim 5, wherein the acceptor is polymer particles filled with a light-emitting compound and a lanthanide compound.

7. The method according to claim 6, wherein the light-emitting compound is selected from olefin compounds, preferably selected from thioxene, dibutanedione compounds, dioxene, enol ether, enamine, 9-alkylene xanthane, 9-alkylene-N-9,10 dihydroacridine, aryl ether alkene, aryl imidazole, lucigenin, and their derivatives, more preferably selected from thioxene and its derivatives.

8. The method according to claim 6 or 7, wherein the lanthanide compound is a europium complex.

9. The method according to claim 5, wherein the acceptor comprises an olefin compound and a metal chelate which are in a non-particle form and are soluble in a water-bearing medium.

10. The method according to any one of claims 5 to 9, wherein the acceptor binds directly or indirectly to a first binding conjugate specific to the target molecules to be detected.

11. The method according to any one of claims 5 to 10, wherein the donor is polymer particles filled with a photosensitive compound which is capable of generating singlet oxygen when excited by a red laser beam.

12. The method according to claim 11, wherein the photosensitive compound is selected from one of methylene blue, rose red, porphyrin, and phthalocyanine.

13. The method according to any one of claims 5 to 12, wherein the donor binds directly or indirectly to a label.

14. The method according to any one of claims 1 to 13, wherein in step (1), the reagents required for the chemiluminescent immunoreaction further comprise a second binding conjugate specific to the target molecules to be detected, wherein preferably, the second binding conjugate specific to the target molecules to be detected binds directly or indirectly to a binding conjugate specific to the label.

15. The method according to claim 14, wherein in step (1), the sample to be detected containing the target molecules to be detected is first mixed with the acceptor reagent and the second binding conjugate specific to the target molecules to be detected, and then mixed with the donor reagent.

16. The method according to any one of claims 1 to 15, wherein in step (2), the mixture to be detected is excited to undergo the chemiluminescence by means of energy and/or an active compound, wherein preferably, the mixture to be detected is irradiated with red excitation light of 600-700 nm so as to be excited to undergo the chemiluminescence.

17. The method according to any one of claims 1 to 16, wherein in step (2), a wavelength that is detected for recording the signal values with respect to the chemiluminescence is 520-620 nm.

18. The method according to any one of claims 1 to 17, wherein the target molecules to be detected are an antigen or an antibody, wherein the antigen is an immunogenic substance, and the antibody is an immunoglobulin which is produced by the body capable of recognizing a unique foreign substance.

19. The method according to any one of claims 1 to 18, wherein the standard substance is used as a positive control.

20. The method according to any one of claims 16 to 19, wherein the method specifically comprises the following steps:
(a1) mixing a sample to be detected suspected of containing an antigen (or antibody) to be detected with an acceptor reagent to obtain a mixture, and subjecting the mixture to a first incubation to obtain a mixed solution; then mixing the mixed solution obtained from the first incubation with a donor reagent to obtain a mixture, and subjecting the mixture to a second incubation to form a mixture to be detected;
(a2) exciting the mixture to be detected successively t times with red excitation light of 600-700 nm to cause the mixture to be detected to undergo chemiluminescence, and recording n times signal values with respect to the chemiluminescence, a wavelength detected being 520-620 nm, wherein an n^{th}-time-recorded signal value with respect to the chemiluminescence is marked as RLUn;
(a3) selecting any two signal values from the signal values with respect to the chemiluminescence recorded in the n times, marking the selected two signal values as RLUm and RLUk, respectively, and marking a growth rate from RLUm to RLUk as A, wherein the growth rate A=(RLUm/RLUk-1)×100%;
(a4) making a standard curve based on a series of known concentrations of positive controls containing target molecules to be detected as well as a growth rate A' from RLUm' to RLUk' read with respect to any two reactions of the positive controls happened in step (a2) and step (a3), wherein the concentrations of the standard substances are lower than a concentration at which a HOOK effect occurs; and
(a5) diluting the sample to be detected and measuring the diluted sample to be detected again, if the growth rate A is greater than a maximum value of the standard curve,
wherein t, n, m, and k are natural numbers greater than 0, and k≤m≤n≤t, n≥2.

21. A system using a chemical luminescence immune analysis and measurement method, comprising:
a reaction device, which is configured to conduct a chemiluminescent immunoreaction;
an excitation and reading device, which is configured to: excite the mixture to be detected successively t times to cause the mixture to be detected to undergo chemiluminescence, and record n times signal values with respect to the chemiluminescence, wherein an n^{th}-time-recorded signal value with respect to the chemiluminescence is marked as RLUn; and select any two signal values from the signal values with respect to the chemiluminescence recorded in the n times, mark the selected two signal values as RLUm and RLUk, respectively, and mark a growth rate from RLUm to RLUk as A; and
a processor, which is configured to: make a standard curve based on a series of known concentrations of standard substances containing target molecules to be detected as well as a growth rate A' from RLUm' to RLUk' read with respect to any two reactions of the standard substances, wherein the concentrations of the standard substances are lower than a concentration at which a HOOK effect occurs,
wherein t, n, m, and k are natural numbers greater than 0, and k≤m≤n≤t, n≥2.

22. The system according to claim 21, wherein a method of using the system comprises the following steps:
(1) mixing and reacting a sample to be detected suspected of containing target molecules to be detected with reagents required for a chemiluminescent immunoreaction to form a mixture to be detected;
(2) exciting the mixture to be detected successively t times to cause the mixture to be detected to undergo chemiluminescence, and recording n times signal values with respect to the chemiluminescence, wherein an n^{th}-time-recorded signal value with respect to the chemiluminescence is marked as RLUn;
(3) selecting any two signal values from the signal values with respect to the chemiluminescence recorded in the n times, marking the selected two signal values as RLUm and RLUk, respectively, and marking a growth rate from RLUm to RLUk as A;
(4) making a standard curve based on a series of known concentrations of standard substances containing the target molecules to be detected as well as a growth rate A' from RLUm' to RLUk' read with respect to any two reactions of the standard substances happened in step (2) and step (3), wherein the concentrations of the standard substances are lower than a concentration at which a HOOK effect occurs; and
(5) diluting the sample to be detected and measuring the diluted sample to be detected again, if the growth rate A is greater than a maximum value of the standard curve,
wherein t, n, m, and k are natural numbers greater than 0, and k≤m≤n≤t, n≥2.

23. The system according to claim 21 or 22, wherein the growth rate A=(RLUm/RLUk-1 )×100%.

24. The system according to any one of claims 21 to 23, wherein n is greater than 2.

25. The system according to any one of claims 22 to 24, wherein in step (1), the chemiluminescent immunoreaction is a homogeneous chemiluminescent immunoreaction.

26. The system according to any one of claims 22 to 25, wherein in step (1), the reagents required for the chemiluminescent immunoreaction comprises an acceptor reagent and a donor reagent, wherein:
the donor reagent comprises a donor which is capable of generating singlet oxygen in an excited state; and
the acceptor reagent comprises an acceptor which is capable of reacting with singlet oxygen to generate a detectable chemiluminescence signal value.

27. The system according to claim 26, wherein the acceptor is polymer particles filled with a light-emitting compound and a lanthanide compound.

28. The system according to claim 27, wherein the light-emitting compound is selected from olefin compounds, preferably selected from thioxene, dibutanedione compounds, dioxene, enol ether, enamine, 9-alkylene xanthane, 9-alkylene-N-9,10 dihydroacridine, aryl ether alkene, aryl imidazole, lucigenin, and their derivatives, more preferably selected from thioxene and its derivatives.

29. The system according to claim 27 or 28, wherein the lanthanide compound is a europium complex.

30. The system according to claim 26, wherein the acceptor comprises an olefin compound and a metal chelate which are in a non-particle form and are soluble in a water-bearing medium.

31. The system according to any one of claims 26 to 30, wherein the acceptor binds directly or indirectly to a first binding conjugate specific to the target molecules to be detected.

32. The system according to any one of claims 26 to 31, wherein the donor is polymer particles filled with a photosensitive compound which is capable of generating singlet oxygen when excited by a red laser beam.

33. The system according to claim 32, wherein the photosensitive compound is selected from one of methylene blue, rose red, porphyrin, and phthalocyanine.

34. The system according to any one of claims 26 to 33, wherein the donor binds directly or indirectly to a label.

35. The system according to any one of claims 22 to 34, wherein in step (1), the reagents required for the chemiluminescent immunoreaction further comprise a second binding conjugate specific to the target molecules to be detected, wherein preferably, the second binding conjugate specific to the target molecules to be detected binds directly or indirectly to a binding conjugate specific to the label.

36. The system according to claim 35, wherein in step (1), the sample to be detected containing the target molecules to be detected is first mixed with the acceptor reagent and the second binding conjugate specific to the target molecules to be detected, and then mixed with the donor reagent.

37. The system according to any one of claims 22 to 36, wherein in step (2), the mixture to be detected is excited to undergo the chemiluminescence by means of energy and/or an active compound, wherein preferably, the mixture to be detected is irradiated with red excitation light of 600-700 nm so as to be excited to undergo the chemiluminescence.

38. The system according to any one of claims 22 to 37, wherein in step (2), a wavelength that is detected for recording the signal values with respect to the chemiluminescence is 520-620 nm.

39. The system according to any one of claims 21 to 38, wherein the target molecules to be detected are an antigen or an antibody, wherein the antigen is an immunogenic substance, and the antibody is an immunoglobulin which is produced by the body capable of recognizing a unique foreign substance.

40. The system according to any one of claims 21 to 39, wherein the standard substance is used as a positive control.

41. The system according to any one of claims 37 to 40, wherein the method specifically comprises the following steps:
(a1) mixing a sample to be detected suspected of containing an antigen (or antibody) to be detected with an acceptor reagent to obtain a mixture, and subjecting the mixture to a first incubation to obtain a mixed solution; then mixing the mixed solution obtained from the first incubation with a donor reagent to obtain a mixture, and subjecting the mixture to a second incubation to form a mixture to be detected;
(a2) exciting the mixture to be detected successively t times with red excitation light of 600-700 nm to cause the mixture to be detected to undergo chemiluminescence, and recording n times signal values with respect to the chemiluminescence, a wavelength detected being 520-620 nm, wherein an n^{th}-time-recorded signal value with respect to the chemiluminescence is marked as RLUn;
(a3) selecting any two signal values from the signal values with respect to the chemiluminescence recorded in the n times, marking the selected two signal values as RLUm and RLUk, respectively, and marking a growth rate from RLUm to RLUk as A, wherein the growth rate A=(RLUm/RLUk-1)×100%;
(a4) making a standard curve based on a series of known concentrations of positive controls containing target molecules to be detected as well as a growth rate A' from RLUm' to RLUk' read with respect to any two reactions of the positive controls happened in step (a2) and step (a3), wherein the concentrations of the standard substances are lower than a concentration at which a HOOK effect occurs; and
(a5) diluting the sample to be detected and measuring the diluted sample to be detected again, if the growth rate A is greater than a maximum value of the standard curve,
wherein t, n, m, and k are natural numbers greater than 0, and k≤m≤n≤t, n≥2.

42. A kit, which comprises reagents required for chemical luminescence immune analysis and measurement, wherein a method of using the kit comprises the following steps:
(1) mixing and reacting a sample to be detected suspected of containing target molecules to be detected with reagents required for a chemiluminescent immunoreaction to form a mixture to be detected;
(2) exciting the mixture to be detected successively t times to cause the mixture to be detected to undergo chemiluminescence, and recording n times signal values with respect to the chemiluminescence, wherein an n^{th}-time-recorded signal value with respect to the chemiluminescence is marked as RLUn;
(3) selecting any two signal values from the signal values with respect to the chemiluminescence recorded in the n times, marking the selected two signal values as RLUm and RLUk, respectively, and marking a growth rate from RLUm to RLUk as A;
(4) making a standard curve based on a series of known concentrations of standard substances containing the target molecules to be detected as well as a growth rate A' from RLUm' to RLUk' read with respect to any two reactions of the standard substances happened in step (2) and step (3), wherein the concentrations of the standard substances are lower than a concentration at which a HOOK effect occurs; and
(5) diluting the sample to be detected and measuring the diluted sample to be detected again, if the growth rate A is greater than a maximum value of the standard curve,
wherein t, n, m, and k are natural numbers greater than 0, and k≤m≤n≤t, n≥2.

43. The kit according to claim 42, wherein the method of using the kit specifically comprises the following steps:
(a1) mixing a sample to be detected suspected of containing an antigen (or antibody) to be detected with an acceptor reagent to obtain a mixture, and subjecting the mixture to a first incubation to obtain a mixed solution; then mixing the mixed solution obtained from the first incubation with a donor reagent to obtain a mixture, and subjecting the mixture to a second incubation to form a mixture to be detected;
(a2) exciting the mixture to be detected successively t times with red excitation light of 600-700 nm to cause the mixture to be detected to undergo chemiluminescence, and recording n times signal values with respect to the chemiluminescence, a wavelength detected being 520-620 nm, wherein an n^{th}-time-recorded signal value with respect to the chemiluminescence is marked as RLUn;
(a3) selecting any two signal values from the signal values with respect to the chemiluminescence recorded in the n times, marking the selected two signal values as RLUm and RLUk, respectively, and marking a growth rate from RLUm to RLUk as A, wherein the growth rate A=(RLUm/RLUk-1)×100%;
(a4) making a standard curve based on a series of known concentrations of standard substances containing target molecules to be detected as well as a growth rate A' from RLUm' to RLUk' read with respect to any two reactions of the standard substances happened in step (2) and step (3), wherein the concentrations of the standard substances are lower than a concentration at which a HOOK effect occurs; and
(a5) diluting the sample to be detected and measuring the diluted sample to be detected again, if the growth rate A is greater than a maximum value of the standard curve,
wherein t, n, m, and k are natural numbers greater than 0, and k≤m≤n≤t, n≥2.

44. The kit according to claim 42 or 43, wherein the growth rate A=(RLUm/RLUk-1)×100%.

45. The kit according to claim 42 or 43, wherein n is greater than 2.

46. Use of the method according to any one of claims 1 to 20, the system according to any one of claims 21 to 41, or the kit according to any one of claims 42 to 45, in AFP detection.
